(19) Europäisches Patentamt
European Patent Office
Office européen des brevets

(11) **EP 4 759 296 A1**

(12) **EUROPEAN PATENT APPLICATION**
published in accordance with Art. 153(4) EPC

(43) Date of publication:
**17.06.2026 Bulletin 2026/25**

(21) Application number: **24851917.5**

(22) Date of filing: **08.08.2024**

(51) International Patent Classification (IPC):
***A61K 8/29*** (2006.01) ***A61K 8/06*** (2006.01)
***A61K 8/19*** (2006.01) ***A61K 8/37*** (2006.01)
***A61K 8/46*** (2006.01) ***A61K 8/81*** (2006.01)
***A61K 8/89*** (2006.01) ***A61K 8/893*** (2006.01)
***A61Q 1/02*** (2006.01)

(52) Cooperative Patent Classification (CPC):
**A61K 8/02; A61K 8/06; A61K 8/19; A61K 8/25; A61K 8/29; A61K 8/37; A61K 8/46; A61K 8/81; A61K 8/89; A61K 8/893; A61K 8/90; A61Q 1/02**

(86) International application number:
**PCT/JP2024/028457**

(87) International publication number:
**WO 2025/033500 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.08.2023 JP 2023131143**

(71) Applicant: **Kao Corporation**
**Tokyo 103-8210 (JP)**

(72) Inventor: **TATEBE, Momoko**
**Tokyo 103-8210 (JP)**

(74) Representative: **Hoffmann Eitle**
**Patent- und Rechtsanwälte PartmbB**
**Arabellastraße 30**
**81925 München (DE)**

(54) **WATER-IN-OIL EMULSION COMPOSITION**

(57) Provided is a water-in-oil emulsion composition for impregnation comprising the following components (A), (B), (C), (D), and (E): (A) a hydrophobized color pigment, (B) an acrylic silicone polymer, (C) one or more members selected from the group consisting of polyether-modified silicone and polyglyceryl ether-modified silicone, (D) spherical silica, and (E) an ester oil which is in a liquid state at 25°C.



Processed by Luminess, 75001 PARIS (FR)

## Description

Field of the Invention

The present invention relates to a water-in-oil emulsion composition.

Background of the Invention

Heretofore, cosmetics have been developed in a form in which a holder, such as sponge, housed in a compact container is impregnated with a liquid cosmetic, and the liquid cosmetic is taken from the holder by finger or an applicator when using to be applied to the skin upon application.
For example, Patent Literature 1 states that a cosmetic product containing a polyether urethane foam impregnated with a cosmetic composition is excellent in stability, portability, and use impression.
Patent Literature 2 states that a cosmetic product having water-insoluble sponge impregnated with a cosmetic composition containing a specific organic ultraviolet shielding agent is excellent in ultraviolet shielding function.
Patent Literature 3 states that a water-in-oil emulsion cosmetic for impregnation containing cross-linked polyether-modified silicone or cross-linked polyglycerin-modified silicone and an acrylic polymer having a dendrimeric siloxane structure in a side chain or silicone-modified pullulan is excellent in use impression and effect of refining skin pores and the like while maintaining appropriate filling to an impregnated material and holdability.

(Patent Literature 1) JP-A-2013-530252
(Patent Literature 2) JP-A-2017-088631
(Patent Literature 3) JP-A-2019-131489

Summary of the Invention

The present invention also relates to a water-in-oil emulsion composition for impregnation comprising the following components (A), (B), (C), (D), and (E):

(A) a hydrophobized color pigment,
(B) an acrylic silicone polymer,
(C) one or more members selected from the group consisting of polyether-modified silicone and polyglyceryl ether-modified silicone,
(D) spherical silica, and
(E) an ester oil which is in a liquid state at 25°C.

The present invention also relates to a cosmetic in which a holder impregnated with the water-in-oil emulsion composition is housed in a compact container.
The present invention also relates to a method for using the cosmetic, characterized by taking a water-in-oil emulsion composition impregnated into a holder by finger or an applicator from the holder, and applying it to the skin.
The present invention also relates to a cosmetic kit comprising a set of a holder impregnated with a water-in-oil emulsion composition and a compact container, the water-in-oil emulsion composition comprising the following components (A), (B), (C), (D), and (E):

(A) a hydrophobized color pigment,
(B) an acrylic silicone polymer,
(C) one or more members selected from the group consisting of polyether-modified silicone and polyglyceryl ether-modified silicone,
(D) spherical silica, and
(E) an ester oil which is in a liquid state at 25°C.

The present invention also relates to a water-in-oil emulsion composition comprising the following components (A), (B), (C), (D), and (E):

(A) a hydrophobized color pigment,
(B) an acrylic silicone polymer,
(C) one or more members selected from the group consisting of polyether-modified silicone and polyglyceryl ether-modified silicone,

(D) spherical silica, and
(E) an ester oil which is in a liquid state at 25°C.

Detailed Description of the Invention

Problems of conventional cosmetics are uneven finish and also poor coverage of uneven skin tone.

The present inventor found that a water-in-oil emulsion composition comprising a hydrophobized color pigment, an acrylic silicone polymer, one or more members selected from the group consisting of polyether-modified silicone and polyglyceryl ether-modified silicone, spherical silica, and a liquid ester oil in combination offers even finish, is excellent in coverage of uneven skin tone, is unlikely to cause smudged makeup, produces glossy finish, and is also excellent in makeup durability.

The water-in-oil emulsion composition used in the present invention offers even finish, is excellent in coverage of uneven skin tone, is unlikely to cause smudged makeup, produces glossy finish, and is also excellent in makeup durability. The water-in-oil emulsion composition is suitable for use as a cosmetic in which a holder impregnated with the water-in-oil emulsion composition is housed in a compact container.

The component (A) used in the present invention is a hydrophobized color pigment.

The color pigment is a color pigment for use in usual cosmetics. Examples thereof include: inorganic pigments including metal oxides such as titanium oxide, zinc oxide, cerium oxide, aluminum oxide, yellow iron oxide, black iron oxide, red iron oxide, iron blue, ultramarine, chromium oxide, and chromium hydroxide, metal complexes such as manganese violet and cobalt titanate, and further, carbon black; synthetic organic pigments, organic dyes, and lake pigments thereof, such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 405, Red No. 505, Orange No. 203, Orange No. 204, Orange No. 205, Yellow No. 4, Yellow No. 5, Yellow No. 401, Blue No. 1, and Blue No. 404; and natural organic dyes such as β-carotene, caramel, and paprika pigment, and include complexes of these color pigments, and mixed pigments of these color pigments diluted with extenders such as barium sulfate or talc.

The color pigment preferably contains a metal oxide, more preferably comprises one or more members selected from the group consisting of titanium oxide, zinc oxide, yellow iron oxide, black iron oxide, and red iron oxide, and further more preferably comprises one or more members selected from the group consisting of titanium oxide, yellow iron oxide, black iron oxide, and red iron oxide, from the viewpoint of covering the skin and imparting aesthetic makeup effect.

The hydrophobization treatment of such a color pigment is not limited as long as the treatment can be practiced for usual cosmetic powders. Dry treatment, wet treatment, or the like can be performed using a surface treatment agent such as a silicone compound, a metal soap, an amino acid compound, lecithin, an oil agent, or organic titanate.

Specific examples of the surface treatment agent include: silicone compounds including alkylalkoxysilane such as methyltrimethoxysilane, ethyltrimethoxysilane, hexyltrimethoxysilane, octyltrimethoxysilane, octyltriethoxysilane, and triethoxycaprylylsilane, dimethylpolysiloxane, methyl hydrogen polysiloxane, cyclic silicone, cross-linked silicone, silicone resin, and acrylic modified silicone; metal soaps such as aluminum stearate, aluminum myristate, zinc stearate, and magnesium stearate; amino acids such as proline, hydroxyproline, alanine, glycine, sarcosine, glutamic acid, aspartic acid, lysine, and their derivatives; acylated amino acids such as stearoyl glutamic acid, lauroyl aspartic acid, dilauramidoglutamide lysine, and lauroyl lysine; lecithin and hydrogenated lecithin; oil agents such as polyisobutylene, waxes, fat/oil, and fatty acids; and organic titanate such as isopropyl triisostearoyl titanate.

The acylated amino acid includes salts of the acylated amino acid. Examples of the salt of the acylated amino acid include Na, Ca, Al, Mg, Zn, Zr, and Ti salts. The salt of the acylated amino acid preferably comprises one or more members selected from the group consisting of Na and Ca salts, and more preferably comprises Na salt. The acylated amino acid salt preferably comprises one or more members selected from the group consisting of disodium stearoyl glutamate and sodium lauroyl aspartate, and more preferably comprises disodium stearoyl glutamate, from the viewpoint of even finish and excellent coverage of uneven skin tone.

The surface treatment agent preferably comprises one or more members selected from the group consisting of a silicone compound, an amino acid, an acylated amino acid, and organic titanate, more preferably comprises one or more members selected from the group consisting of alkylalkoxysilane, an amino acid, and an acylated amino acid, further more preferably comprises one or more members selected from the group consisting of alkylalkoxysilane, disodium stearoyl glutamate, sodium lauroyl aspartate, and isopropyl triisostearoyl titanate, even more preferably comprises one or more members selected from the group consisting of alkylalkoxysilane, disodium stearoyl glutamate, and isopropyl triisostearoyl titanate, and is even more preferably disodium stearoyl glutamate, from the viewpoint of even finish and excellent coverage of uneven skin tone.

The hydrophobization treatment can be performed in accordance with a usual method.

The amount of the treatment agent in the hydrophobization treatment is preferably from 0.1 to 15% by mass, more preferably from 1 to 12% by mass, based on the powder to be coated from the viewpoint of improvement in dispersibility in a solvent.

One of or two or more in combination of these components (A) can be used. The content is preferably 1% by mass or more, more preferably 3% by mass or more, further more preferably 5% by mass or more, and preferably 30% by mass or less, more preferably 25% by mass or less, further more preferably 20% by mass or less, in the entire composition of the water-in-oil emulsion composition from the viewpoint of even finish and excellent coverage of uneven skin tone. The content of the component (A) is preferably from 1 to 30% by mass, more preferably from 3 to 25% by mass, further more preferably from 5 to 20% by mass, in the entire composition of the water-in-oil emulsion composition.

Examples of the acrylic silicone polymer as the component (B) used in the present invention include vinyl polymers having a carbosiloxane dendrimer structure in a side chain, and acryl-silicone graft copolymers.

In the vinyl polymer having a carbosiloxane dendrimer structure in a side chain, the carbosiloxane dendrimer structure is preferably a group of the following formula (1) from the viewpoint of forming a soft coating film:

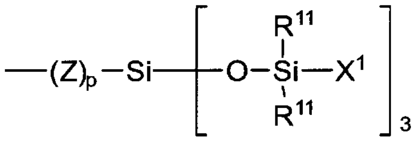

(1)

In the formula, Z is a divalent organic group, p is 0 or 1, and $R^{11}$ is an alkyl group or an aryl group each having 1 to 10 carbon atoms. A plurality of $R^{11}$ moieties may be the same or different.

$X^1$ is a silylalkyl group of the following formula at i = 1:

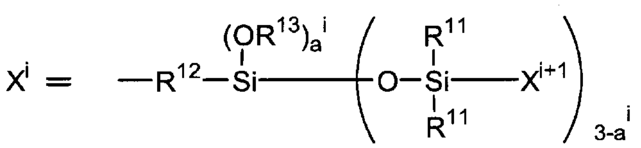

In the formula, $R^{11}$ is as defined above, $R^{12}$ is an alkylene group having 2 to 10 carbon atoms, and $R^{13}$ is an alkyl group having 1 to 10 carbon atoms. A plurality of $R^{11}$ moieties may be the same or different.

$X^{i+1}$ is a group selected from the group consisting of a hydrogen atom, an alkyl group, an aryl group, and the silylalkyl group each having 1 to 10 carbon atoms. i is an integer of from 1 to 10 which indicates a generation of the silylalkyl group, and $a^i$ is an integer of from 0 to 3.

In the formula (1), Z is a divalent organic group. Examples thereof include alkylene groups, arylene groups, aralkylene groups, ester-containing divalent organic groups, ether-containing divalent organic groups, ketone-containing divalent organic groups, and amide group-containing divalent organic groups. Among them, an organic group of any of the following formulas is preferred.

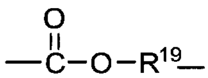

(i)

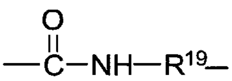

(ii)

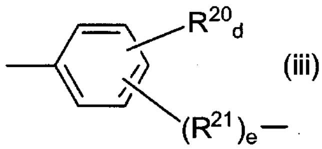

(iii)

In the formulas, $R^{19}$ is an alkylene group having 1 to 10 carbon atoms. Examples thereof include a methylene group, an ethylene group, a propylene group, and a butylene group. Among them, an ethylene group or a propylene group is preferred. $R^{20}$ is an alkyl group having 1 to 10 carbon atoms. Examples thereof include a methyl group, an ethyl group, a propyl group, and a butyl group. Among them, a methyl group is preferred. $R^{21}$ is an alkylene group having 1 to 10 carbon atoms. Examples thereof include alkylene groups such as a methylene group, an ethylene group, a propylene group, and a butylene group. Among them, an ethylene group is preferred. d is an integer of from 0 to 4, and e is 0 or 1.

In the formula (1), $R^{11}$ is an alkyl group or an aryl group each having 1 to 10 carbon atoms. Examples of the alkyl group include a methyl group, an ethyl group, a propyl group, a butyl group, a pentyl group, an isopropyl group, an isobutyl

group, a cyclopentyl group, and a cyclohexyl group. Examples of the aryl group include a phenyl group and a naphthyl group. Among them, a methyl group or a phenyl group is preferred, and a methyl group is more preferred.

$X^1$ is a silylalkyl group of the following formula at i = 1:

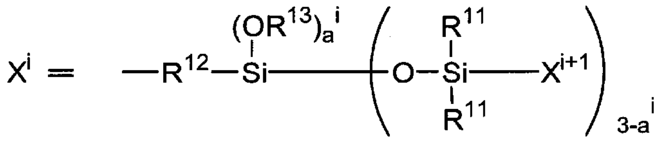

In the formula, $R^{11}$ is as defined above. $R^{12}$ is an alkylene group having 2 to 10 carbon atoms. Examples thereof include: linear alkylene groups such as an ethylene group, a propylene group, a butylene group, and a hexylene group; and branched alkylene groups such as a methylmethylene group, a methylethylene group, a 1-methylpentylene group, and a 1,4-dimethylbutylene group. Among them, an ethylene group, a methylethylene group, a hexylene group, a 1-methylpentylene group, or a 1,4-dimethylbutylene group is preferred. $R^{13}$ is an alkyl group having 1 to 10 carbon atoms. Examples thereof include a methyl group, an ethyl group, a propyl group, a butyl group, and an isopropyl group. $X^{i+1}$ is a group selected from the group consisting of a hydrogen atom, an alkyl group, an aryl group, and the silylalkyl group each having 1 to 10 carbon atoms. $a^i$ is an integer of from 0 to 3. i is an integer of from 1 to 10, which indicates the number of generations of the silylalkyl group, i.e., the number of repeats of the silylalkyl group.

The vinyl polymer having a carbosiloxane dendrimer structure in a side chain is preferably a vinyl polymer having a carbosiloxane dendrimer structure prepared by (co)polymerizing from 0 to 99.9 parts by mass of a vinyl monomer (B1) other than (B2) and from 100 to 0.1 parts by mass of a carbosiloxane dendrimer (B2) represented by the formula (2) and having a radical-polymerizable organic group:

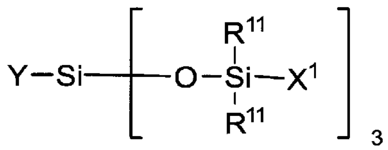

(2)

wherein Y is a radical-polymerizable organic group, and $R^{11}$ and $X^1$ are as defined above.

In the formula, Y is a radical-polymerizable organic group, and $R^{11}$ is an alkyl group or an aryl group each having 1 to 10 carbon atoms. Examples of the alkyl group include a methyl group, an ethyl group, a propyl group, a butyl group, a pentyl group, an isopropyl group, an isobutyl group, a cyclopentyl group, and a cyclohexyl group. Examples of the aryl group include a phenyl group and a naphthyl group. Among them, a methyl group or a phenyl group is preferred, and a methyl group is more preferred.

In this vinyl polymer, the vinyl monomer as the component (B1) may not be particularly limited by its type or the like as long as it has a radical-polymerizable vinyl group. Examples of such a vinyl monomer include: lower alkyl (meth)acrylate such as methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, n-hexyl (meth)acrylate, and cyclohexyl (meth)acrylate; higher alkyl (meth)acrylate such as 2-ethylhexyl (meth)acrylate, octyl (meth)acrylate, lauryl (meth)acrylate, and stearyl (meth)acrylate; fatty acid vinyl ester such as vinyl acetate, vinyl propionate, vinyl butyrate, vinyl caproate, vinyl 2-ethylhexanoate, vinyl laurate, and vinyl stearate; aromatic group-containing monomers such as styrene, vinyltoluene, benzyl (meth)acrylate, and phenoxyethyl (meth)acrylate; amide group-containing vinyl monomers such as (meth)acrylamide, N-methylol(meth)acrylamide, N-methoxymethyl(meth)acrylamide, isobutoxymethoxy(meth)acrylamide, N,N-dimethyl(meth)acrylamide, vinylpyrrolidone, and N-vinylacetamide; hydroxy group-containing vinyl monomers such as hydroxyethyl (meth)acrylate, hydroxypropyl (meth)acrylate, glyceryl (meth)acrylate, and hydroxyethylacrylamide; carboxylic acid-containing vinyl monomers such as (meth)acrylic acid, itaconic acid, crotonic acid, fumaric acid, and maleic acid, and their salts; ether bond-containing vinyl monomers such as tetrahydrofurfuryl (meth)acrylate, butoxyethyl (meth)acrylate, ethoxy diethylene glycol (meth)acrylate, polyethylene glycol (meth)acrylate, polypropylene glycol mono(meth)acrylate, hydroxy butyl vinyl ether, cetyl vinyl ether, and 2-ethyl hexyl vinyl ether; reactive group-containing monomers such as glycidyl (meth)acrylate, (meth)allyl glycidyl ether, methacryloyloxyethyl isocyanate, and (meth)acryloxypropyltrimethoxysilane; macromonomers such as polydimethylsiloxane containing a (meth)acrylic group at one end, and polydimethylsiloxane containing a styryl group at one end; butadiene; vinyl chloride; vinylidene chloride; (meth)acrylonitrile; dibutyl fumarate; maleic anhydride; radical-polymerizable unsaturated monomers having a sulfonic acid group such as styrenesulfonic acid and acrylamido-2-methylpropanesulfonic acid, and their alkali metal salts, ammonium salts, and organic amine salts; quaternary ammonium salts derived from (meth)acrylic acid, such as 2-hydroxy-3-methacryloxypropyl trimethylammonium chloride; and methacrylic acid ester of alcohol having a tertiary amino

group, such as diethylaminoethyl methacrylate, vinylpyridine, and their quaternary ammonium salts.

A polyfunctional vinyl monomer may be used. Examples thereof include trimethylolpropane tri(meth)acrylate, pentaerythritol tri(meth)acrylate, ethylene glycol di(meth)acrylate, tetraethylene glycol di(meth)acrylate, polyethylene glycol di(meth)acrylate, 1,4-butanediol di(meth)acrylate, 1,6-hexanediol di(meth)acrylate, neopentyl glycol di(meth)acrylate, trimethylolpropane trioxyethyl (meth)acrylate, tris(2-hydroxyethyl) isocyanurate di(meth)acrylate, tris(2-hydroxyethyl) isocyanurate tri(meth)acrylate, and unsaturated group-containing silicone compounds such as styryl group-terminated polydimethylsiloxane.

The carbosiloxane dendrimer as the component (B2) may not be particularly limited by its type or the like as long as it has a radical-polymerizable organic group of the formula (2). In the formula (2), Y is a radical-polymerizable organic group and may not be limited as long as it is a radical-reactable organic group. Specific examples thereof include (meth)acryloxy group-containing organic groups, (meth)acrylamide group-containing organic groups, and styryl group-containing organic groups each represented by the following formulas, and alkenyl groups having 2 to 10 carbon atoms:

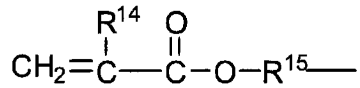

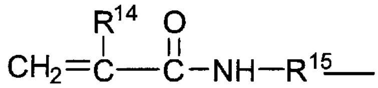

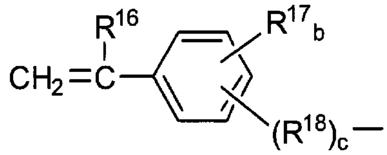

wherein each of $R^{14}$ and $R^{16}$ is a hydrogen atom or a methyl group, each of $R^{15}$ and $R^{18}$ is an alkylene group having 1 to 10 carbon atoms, $R^{17}$ is an alkyl group having 1 to 10 carbon atoms, b is an integer of from 0 to 4, and c is 0 or 1.

Examples of such a radical-polymerizable organic group include a 2-acryloyloxyethyl group, a 3-acryloyloxypropyl group, a 2-methacryloyloxyethyl group, a 3-methacryloyloxypropyl group, a 4-vinylphenyl group, a 3-vinylphenyl group, a 4-(2-propenyl)phenyl group, a 3-(2-propenyl)phenyl group, a 2-(4-vinylphenyl)ethyl group, a 2-(3-vinylphenyl)ethyl group, a vinyl group, an allyl group, a methallyl group, and a 5-hexenyl group.

In the formula (2), the carbosiloxane dendrimer as the component (B2) at i = 1, i.e., when the number of generations of the silylalkyl group is 1, is of the formula:

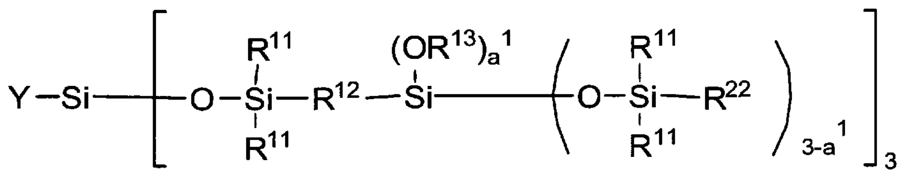

wherein Y, $R^{11}$, $R^{12}$, and $R^{13}$ are as defined above, $R^{22}$ is a hydrogen atom or is the same as $R^{11}$, and $a^1$ is the same as $a^i$, though an average total number of $a^1$ in one molecule is from 0 to 7.

Examples of the carboxy dendrimer (B2) containing such a radical-polymerizable organic group include carbosiloxane dendrimers of the following average compositional formulas:

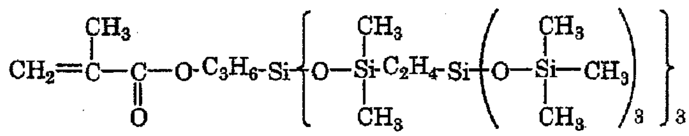

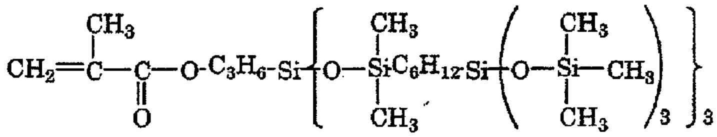

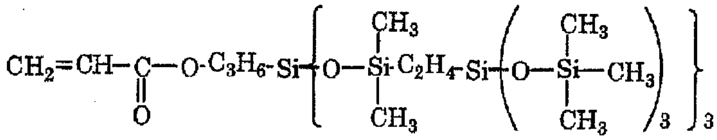

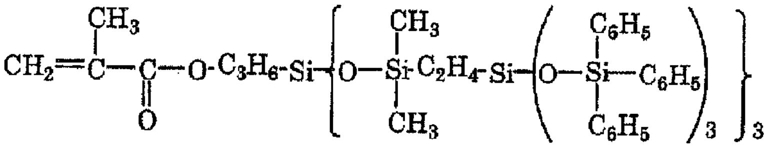

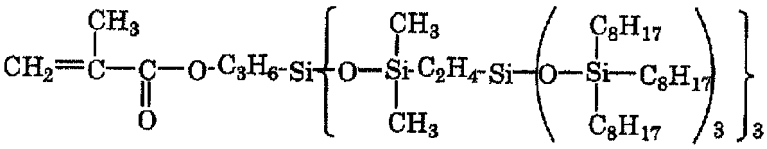

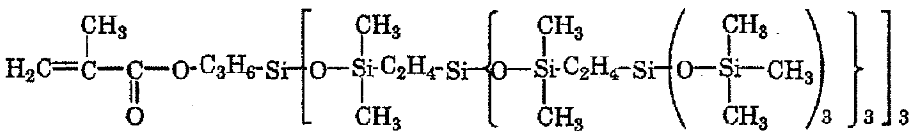

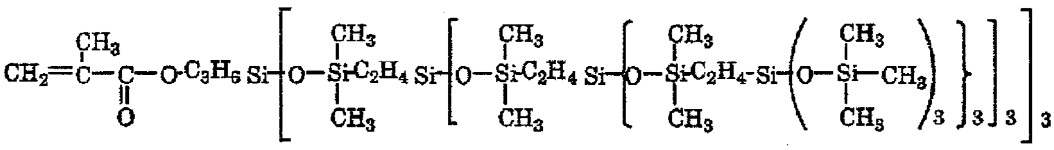

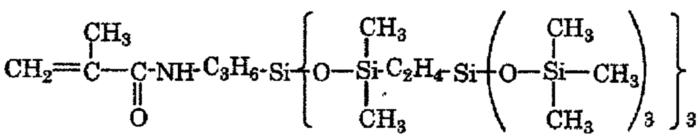

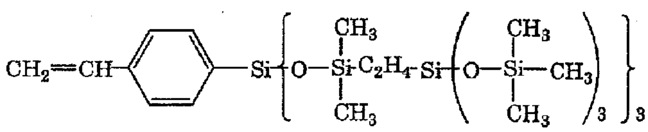

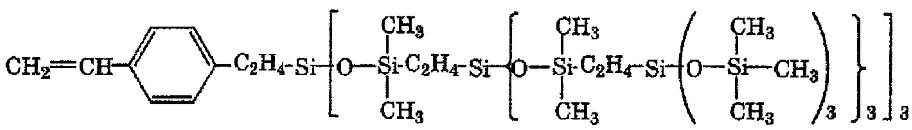

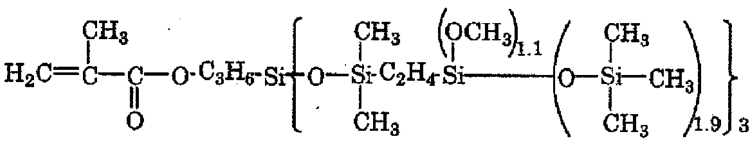

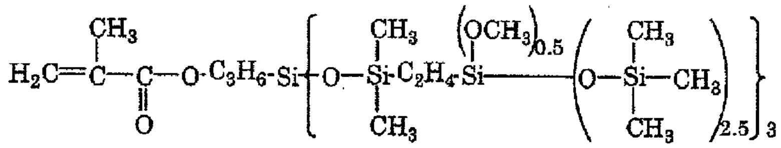

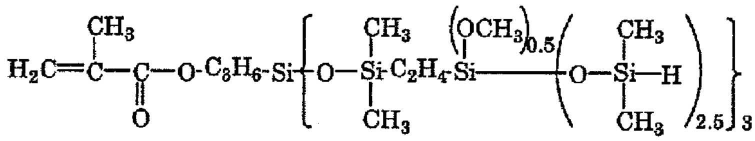

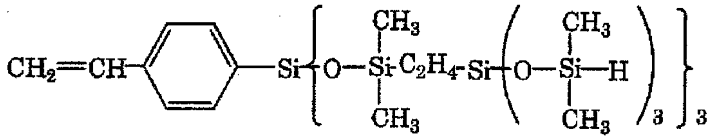

The component (B2) is preferably a carbosiloxane dendrimer of the following average compositional formula from the viewpoint of forming a soft coating film:

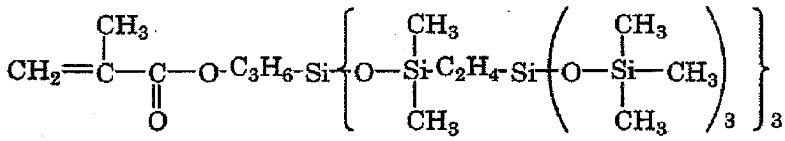

Such a carbosiloxane dendrimer can be produced in accordance with a production method described in, for example, JP-A-11-1530 or JP-A-2000-63225.

In the vinyl polymer having a dendrimer structure used in the present invention, the mass ratio between the component (B1) and the component (B2) ((B1):(B2)) is in the range of preferably from 0:100 to 99.9:0.1, more preferably from 5:95 to 90:10, further more preferably from 10:90 to 80:20.

The number-average molecular weight of the vinyl polymer having a carbosiloxane dendrimer structure used in the present invention is preferably from 3 000 to 2 000 000, more preferably from 5 000 to 800 000, from the viewpoint of forming a soft coating film.

Its nature may be any of liquid, gum, paste, and solid states at 25°C and is preferably a solid state from the viewpoint of forming a soft coating film. A solution or a dispersion obtained by dilution with a solvent is preferred from the viewpoint of conformability to the skin and formation of a coating film. Among others, a dispersion obtained by dilution with a liquid oil is preferably used. One or more members selected from the group consisting of a silicone oil and a hydrocarbon oil are preferably used, one or more members selected from the group consisting of dimethylpolysiloxane, decamethylcyclopentasiloxane, and isododecane are more preferably used, one or more members selected from the group consisting of dimethylpolysiloxane and isododecane are further more preferably used, and dimethylpolysiloxane is even more preferred.

The vinyl polymer having a carbosiloxane dendrimer structure in a side chain is preferably a silicone dendrimer-acryl copolymer and is preferably a nomenclature of cosmetic product "(acrylates/polytrimethylsiloxy methacrylate) copolymer" (INCI name "Acrylates/Polytrimethylsiloxymethacrylate Copolymer"). A commercially available product can be used, such as FA4001CM (solution with 30% by mass in decamethylcyclopentasiloxane), FA4002ID (solution with 40% by mass in isododecane), or DOWSIL FA 4003 DM Silicone Acrylate (solution with 40% by mass in methylpolysiloxane (2 cs)) (all manufactured by Dow Toray Co., Ltd.) dissolved in a solvent in advance.

The acryl-silicone graft copolymer is preferably an acryl-silicone graft copolymer obtained by polymerizing a monomer (B3) comprising a dimethylpolysiloxane compound represented by the formula (3) and having a radical-polymerizable group at one end of a molecular chain, from the viewpoint of excellent coating film formability and softness:

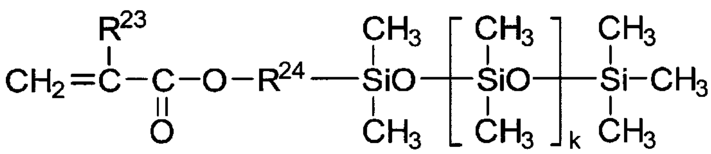

(3)

wherein $R^{23}$ represents a methyl group or a hydrogen atom, $R^{24}$ represents a divalent saturated hydrocarbon group having 1 to 10 carbon atoms, which is optionally terminated with one or two ether bonds, and k represents a number of from 3 to 300.

In the formula (3), the divalent saturated hydrocarbon group having 1 to 10 carbon atoms, which is optionally terminated with one or two ether bonds, represented by $R^{24}$ may be linear or branched and is preferably a group having 1 to 7 carbon atoms. Specific examples thereof include $-CH_2-$, $-(CH_2)_3-$, $-(CH_2)_6-$, $-(CH_2)_8-$, $-(CH_2)_{10}-$, $-CH_2-CH(CH_3)-CH_2-$, $-CH_2CH_2OCH_2CH_2CH_2-$, $-CH_2CH_2OCH_2CH(CH_3)CH_2-$, and $-CH_2CH_2OCH_2CH_2OCH_2CH_2CH_2-$.

k is a number of from 3 to 300 and is preferably from 5 to 100 from the viewpoint of excellent adhesiveness of the emulsion composition to the skin upon application.

Any of compounds given below can be suitably used as the dimethylpolysiloxane compound of the formula (3) from the viewpoint of excellent coating film formability and softness. In the formulas, Me represents a methyl group.

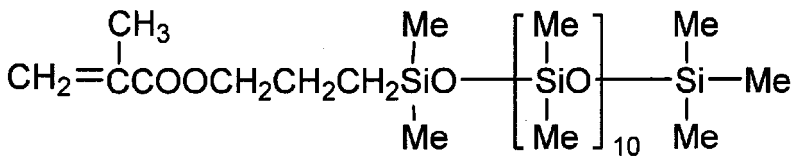

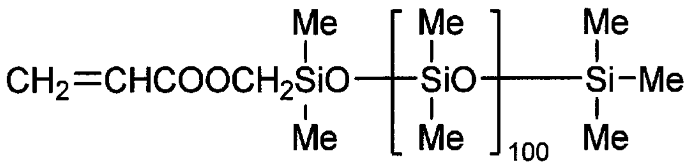

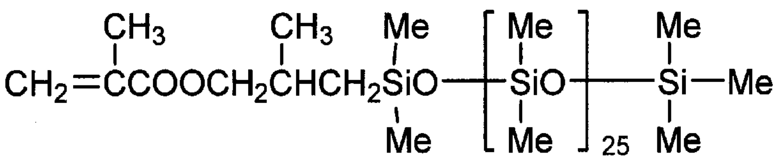

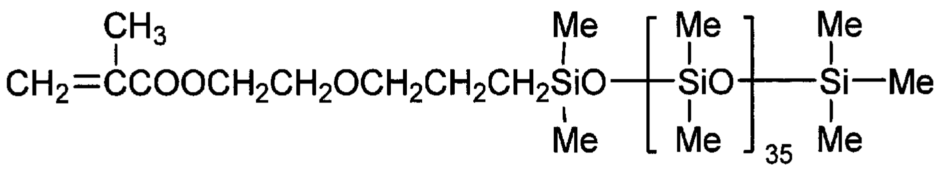

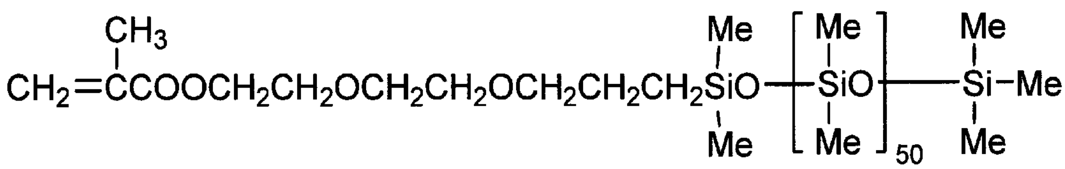

The acryl-silicone graft copolymer is obtained by polymerizing a monomer comprising such a dimethylpolysiloxane compound (B3) of the formula (3). A monomer which may be used in addition to the dimethylpolysiloxane compound (B3) includes a radical-polymerizable monomer (B4) composed mainly of acrylate and/or methacrylate.

The radical-polymerizable monomer composed mainly of acrylate and/or methacrylate is a compound having one radical-polymerizable unsaturated bond in a molecule. The phrase "composed mainly of acrylate and/or methacrylate" means that the total amount of acrylate and/or methacrylate in the radical-polymerizable monomer (B4) accounts for 50% by mass or more of the whole radical-polymerizable monomer.

Examples of the acrylate and/or methacrylate used include: alkyl (meth)acrylate such as methyl (meth)acrylate, ethyl (meth)acrylate, butyl (meth)acrylate, and 2-ethylhexyl (meth)acrylate; hydroxyalkyl (meth)acrylate such as 2-hydroxyethyl (meth)acrylate and 2-hydroxypropyl (meth)acrylate; and perfluoroalkyl (meth)acrylate having 1 to 10 carbon atoms. Among them, methyl methacrylate, butyl methacrylate, or 2-ethylhexyl acrylate is preferred, and a combination of methyl methacrylate, butyl methacrylate, and 2-ethylhexyl acrylate is more preferred.

Examples of the monomer other than acrylate and/or methacrylate include styrene, substituted styrene, vinyl acetate, (meth)acrylic acid, maleic anhydride, maleic acid ester, fumaric acid ester, vinyl chloride, vinylidene chloride,

ethylene, propylene, butadiene, acrylonitrile, and olefin fluoride.

The monomer comprising the dimethylpolysiloxane compound (B3) of the formula (3) can be polymerized in accordance with a usual radical polymerization method, for example, solution polymerization, emulsion polymerization, suspension polymerization, or bulk polymerization, in the presence of a radical polymerization initiator.

The weight-average molecular weight of the resulting acryl-silicone graft copolymer is preferably from 3 000 to 200 000, more preferably from 5 000 to 100 000.

The acryl-silicone graft copolymer is preferably a copolymer obtained by the radical copolymerization of the dimethylpolysiloxane compound (B3) of the formula (3) and the radical-polymerizable monomer (B4) composed mainly of acrylate and/or methacrylate.

In this case, the polymerization ratio of the dimethylpolysiloxane compound (B3) of the formula (3) to the radical-polymerizable monomer (B4) composed mainly of acrylate and/or methacrylate, (B3)/(B4), is preferably from 1/19 to 2/1.

The acryl-silicone graft copolymer is preferably in a solid state at 25°C from the viewpoint of excellent coating film formability and softness.

The acryl-silicone graft copolymer is preferably used after being dissolved in a solvent, from the viewpoint of easiness to spread and excellent coating film formability and softness. Examples of the solvent include silicone oils and hydrocarbon oils. One or more members selected from the group consisting of methyl trimethicone, methylpolysiloxane (2 cs), decamethylcyclopentasiloxane, and isododecane are preferred, one or more members selected from the group consisting of methyl trimethicone, methylpolysiloxane (2 cs), and decamethylcyclopentasiloxane are further more preferred, and methylpolysiloxane (2 cs) is even more preferred.

The acryl-silicone graft copolymer is preferably a nomenclature of cosmetic product "(acrylates/dimethicone) copolymer" (INCI name "Acrylates/Dimethicone Copolymer"). A commercially available product can be used, such as KP-545 (solution with 30% by mass in decamethylcyclopentasiloxane), KP-545L (solution with 40% by mass in methyl polysiloxane (2 cs)), KP-549 (solution with 40% by mass in methyl trimethicone), or KP-550 (solution with 40% by mass in isododecane) (all manufactured by Shin-Etsu Chemical Co., Ltd.) dissolved in a solvent in advance.

For example, KP-578 (manufactured by Shin-Etsu Chemical Co., Ltd.) which is an (acrylates/ethylhexyl acrylate/dimethicone methacrylate) copolymer can also be preferably used.

The component (B) is preferably an acryl-silicone graft copolymer from the viewpoint of the softness of a coating film, excellent evenness of finish, and the suppression of smudged makeup.

One or more of these components (B) can be used. The content is preferably 0.01% by mass or more, more preferably 0.1% by mass or more, further more preferably 0.3% by mass or more, and preferably 5% by mass or less, more preferably 3% by mass or less, further more preferably 1% by mass or less, in the entire composition of the water-in-oil emulsion composition from the viewpoint of the evenness of finish, excellent coverage of uneven skin tone, and the suppression of smudged makeup. The content of the component (B) is preferably from 0.01 to 5% by mass, more preferably from 0.1 to 3% by mass, further more preferably from 0.3 to 1% by mass, in the entire composition of the water-in-oil emulsion composition.

The component (C) used in the present invention is one or more members selected from the group consisting of polyether-modified silicone and polyglyceryl ether-modified silicone.

The polyether-modified silicone is a compound having a methylsilicone chain as the backbone and a side chain with a polyoxyethylene group. Examples thereof include compounds of the formula (4):

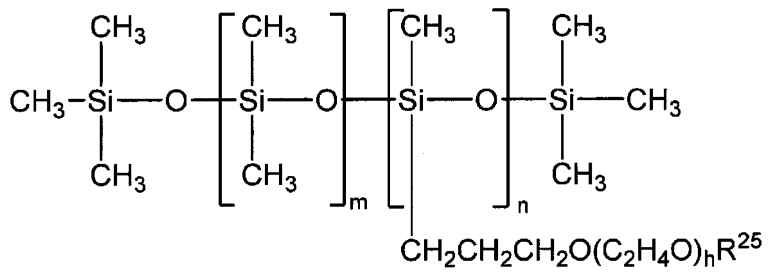

**(4)**

wherein $R^{25}$ represents an alkyl group having 1 to 4 carbon atoms, m represents a number of from 5 to 50, n represents a number of from 2 to 10, and h represents a number of from 5 to 50.

Examples of the polyether-modified silicone include PEG-8 dimethicone, PEG-10 dimethicone, PEG-12 dimethicone, and PEG-14 dimethicone.

The polyglyceryl ether-modified silicone as the component (C) is preferably modified silicone containing an alkyl group having 10 or more carbon atoms and a polyglyceryl group, and has a siloxane skeleton, an alkyl group having 10 or more carbon atoms, and a polyoxyalkylene group or a polyglyceryl group.

The siloxane skeleton refers to a so-called silicone chain. Examples thereof include dimethylpolysiloxane

(dimethicone), methylphenylpolysiloxane, and methyl hydrogen polysiloxane. The silicone chain may not be particularly limited by its length as long as the length can be usually used in cosmetics and the like.

The polyglyceryl ether-modified silicone may be a block copolymer comprising a polyoxyalkylene group or polyglyceryl group chain and a silicone chain in the backbone, may be terminally modified type, or may be side chain modified type in which a polyoxyalkylene group or a polyglyceryl group is bonded to a side chain of the siloxane skeleton.

Oxyalkylene of the polyoxyalkylene group contained in the modified silicone has one or more of ethylene oxide and propylene oxide in one molecule, and preferably has ethylene oxide and propylene oxide. The number of moles of oxyalkylene added in the polyoxyalkylene group is preferably from 4 to 12, more preferably from 8 to 10. The number of moles of glycerin added in the polyglyceryl group contained in the modified silicone is preferably from 1 to 10, more preferably from 1 to 4.

The HLB of the polyglyceryl ether-modified silicone is preferably from 2 to 7, more preferably from 2 to 5, from the viewpoint of suppressing smudged makeup and improving makeup durability.

In the present invention, the HLB (hydrophilic-lipophilic balance) refers to the proportion of the molecular weight of a hydrophilic group moiety to the total molecular weight of a surfactant, and is determined in accordance with the following Griffin's equation.

HLB = 20 × (Molecular weight of a hydrophilic group / Total molecular weight)

Specific examples of the polyglyceryl ether-modified silicone include lauryl PEG-10 tris(trimethylsiloxy)silylethyl dimethicone, cetyl diglyceryl tris(trimethylsiloxy)silylethyl dimethicone, cetyl PEG/PPG-10/1 dimethicone, lauryl PEG-9 polydimethylsiloxyethyl dimethicone, and lauryl PEG/PPG-18/18 methicone.

Examples of the polyglyceryl ether-modified silicone include alkyl glyceryl ether-modified silicone of the following formula (5):

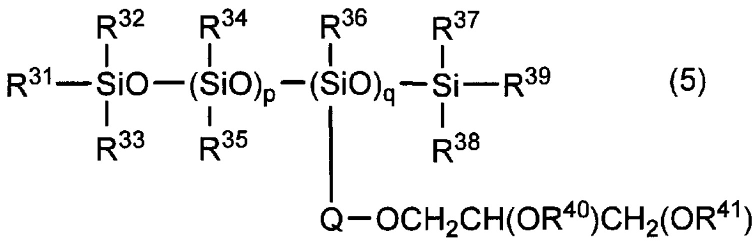

(5)

wherein $R^{31}$ and $R^{39}$ are the same or different and are each independently a linear or branched hydrocarbon group having 1 to 32 carbon atoms in terms of a mode value; $R^{32}$ to $R^{38}$ are the same or different and are each independently a linear or branched hydrocarbon group having 1 to 5 carbon atoms; Q is a linear or branched divalent hydrocarbon group having 3 to 20 carbon atoms; $R^{40}$ and $R^{41}$ are each independently a hydrogen atom or a linear or branched hydrocarbon group having 1 to 28 carbon atoms, and at least one of the moieties is a hydrogen atom; p represents the number of repeat units and is a number of 5 or more and 60 or less as an average value; q represents the number of repeat units and is a number of 2.5 or more and 10 or less as an average value; and constituent units with the number of repeats, p and q, are any of block copolymers and random copolymers, provided that the formula has one or more alkyl groups having 10 or more carbon atoms.

In the formula (5), $R^{31}$ and $R^{39}$ are each independently a linear or branched hydrocarbon group having 1 to 32 carbon atoms in terms of a mode value. In this context, the mode value of the number of carbon atoms refers to the number of carbon atoms in the most abundant hydrocarbon group in a hydrocarbon group chain length having a distribution. The mode value of the number of carbon atoms is preferably from 8 to 32, more preferably from 12 to 28, further more preferably from 16 to 18.

In the formula (5), $R^{32}$ to $R^{38}$ each independently represent a linear or branched hydrocarbon group having 1 to 5 carbon atoms, and may be the same or different. Use of the hydrocarbon group having the number of carbon atoms within this range can effectively prevent a coating film from being plasticized due to an oil agent contained in food. Specific examples thereof include: linear alkyl groups such as a methyl group, an ethyl group, an n-propyl group, an n-butyl group, and a pentyl group; and branched alkyl groups such as an isopropyl group, a sec-butyl group, a tert-butyl group, and a neopentyl group. Among them, a methyl group is preferred from the viewpoint of easy availability.

In the formula (5), Q represents a linear or branched divalent hydrocarbon group having 3 to 20 carbon atoms.

Examples of the divalent hydrocarbon group having 3 to 20 carbon atoms represented by Q include: linear alkylene groups such as a trimethylene group, a tetramethylene group, a pentamethylene group, a hexamethylene group, a heptamethylene group, an octamethylene group, a nonamethylene group, a decamethylene group, an undecamethylene group, a dodecamethylene group, a tridecamethylene group, a tetradecamethylene group, a hexadecamethylene

group, and an octadecamethylene group; and branched alkylene groups such as a propylene group, a 2-methyltetramethylene group, a 2-methylpentamethylene group, a 3-methylpentamethylene group, and a 2-ethyloctamethylene group. Among them, a nonamethylene group, a decamethylene group, an undecamethylene group, a dodecamethylene group, or a tridecamethylene group is preferred.

$R^{40}$ and $R^{41}$ are each independently a hydrogen atom or a linear or branched hydrocarbon group having 1 to 28 carbon atoms, and at least one of the moieties is a hydrogen atom. Examples of the linear or branched hydrocarbon group having 1 to 28 carbon atoms include: linear alkyl groups such as a methyl group, an ethyl group, a propyl group, a butyl group, a pentyl group, a hexyl group, an octyl group, a decyl group, a dodecyl group, a tetradecyl group, a hexadecyl group, an octadecyl group, an eicosyl group, a doeicosyl group, a tetraeicosyl group, a hexaeicosyl group, and an octaeicosyl group; and branched alkyl groups such as an isopropyl group, a sec-butyl group, a tert-butyl group, a neopentyl group, a 1-ethylpropyl group, and a 1-heptyldecyl group. Among them, preferably, both of $R^{40}$ and $R^{41}$ are hydrogen atoms.

In the formula (5), p represents the number of repeat units and represents a number of from 5 to 60, preferably a number of from 20 to 30, as an average value. In this context, the average value of p is calculated from the intensity ratio between peaks attributed to $R^{14}$ and $R^{15}$ by $^{1}$H-NMR on the basis of terminal methyl groups of hydrocarbon groups introduced to both ends of polysiloxane.

q represents the number of repeat units and represents a number of from 2.5 to 10, preferably a number of from 3.0 to 6.0, as an average value. In this context, the average value of q is calculated from the intensity ratio between peaks attributed to methine and methylene hydrogens attached by $OR^{20}$ and $OR^{21}$ by $^{1}$H-NMR on the basis of terminal methyl groups of hydrocarbon groups introduced to both ends of polysiloxane.

The constituent units with the numbers of repeats, p and q, may be block copolymers or random copolymers and are preferably random copolymers. Preferably, p is larger than q.

The alkyl glyceryl ether-modified silicone of the formula (5) can be produced in accordance with a method described in, for example, JP-A-4-134013, through the hydrosilylation reaction of organo hydrogen polysiloxane having at least one silicon-hydrogen bond with corresponding alkenyl glyceryl ether or the like.

The alkyl glyceryl ether-modified silicone of the formula (5) is preferably glyceryl undecyl dimethicone or bisalkyl (C16-18) glyceryl undecyl dimethicone from the viewpoint of forming a network structure between moisture present on the skin and the ester oil as the component (D) during or after application so that a coating film unlikely to cause makeup wearing-off is formed on the skin, thereby suppressing smudged makeup, improving makeup durability, and also improving the evenness of finish.

The component (C) preferably comprises polyglyceryl ether-modified silicone.

One of or two or more in combination of these components (C) can be used. The content is preferably 0.1% by mass or more, more preferably 1% by mass or more, further more preferably 2% by mass or more, and preferably 12% by mass or less, more preferably 7% by mass or less, further more preferably 5% by mass or less, in the entire composition of the water-in-oil emulsion composition from the viewpoint of suppressing smudged makeup and improving makeup durability. The content of the component (C) is preferably from 0.1 to 12% by mass, more preferably from 1 to 7% by mass, further more preferably from 2 to 5% by mass, in the entire composition of the water-in-oil emulsion composition.

In the spherical silica as the component (D), the spherical shape includes a perfect sphere, a substantial sphere, and a spheroid. A spherical powder or the like having surface asperities may be used.

The spherical silica as the component (D) preferably has a specific surface area of 150 $m^2/g$ or less, more preferably 60 $m^2/g$ or less, further more preferably 10 $m^2/g$ or less, and is even more preferably nonporous, from the viewpoint of even finish, excellent coverage of uneven skin tone, the suppression of smudged makeup, and the obtainment of glossy finish.

In the present invention, the specific surface area is measured by a BET method (N2).

The volume-average particle size of the spherical silica as the component (D) is preferably from 1 to 50 μm, more preferably from 1 to 35 μm, further more preferably from 2 to 25 μm, even more preferably from 3 to 15 μm, from the viewpoint of even finish, excellent coverage of uneven skin tone, the suppression of smudged makeup, and the obtainment of glossy finish.

In the present invention, the volume-average particle size is a value measured with ethanol as a dispersion medium using a laser diffraction/scattering particle size distribution analyzer (manufactured by Seishin Enterprise Co., Ltd., LMS-350). The volume-average particle size is an average particle size based on a volume and is a 50% median diameter.

The silica as the component (D) may be used directly or may be used after being hydrophobized by a usual method, as in the component (A).

One of or two or more in combination of these components (D) can be used. The content is preferably 0.1% by mass or more, more preferably 1% by mass or more, further more preferably 2% by mass or more, and preferably 20% by mass or less, more preferably 12% by mass or less, further more preferably 8% by mass or less, in the entire composition of the water-in-oil emulsion composition from the viewpoint of even finish, excellent coverage of uneven skin tone, the suppression of smudged makeup, and the obtainment of glossy finish. The content of the component (D) is preferably from

0.1 to 20% by mass, more preferably from 1 to 12% by mass, further more preferably from 2 to 8% by mass, in the entire composition of the water-in-oil emulsion composition.

In the present invention, the mass ratio of the component (A) to the component (D), (A)/(D), is preferably 0.3 or more, more preferably 0.5 or more, further more preferably 1 or more, and preferably 15 or less, more preferably 7 or less, further more preferably 5 or less, from the viewpoint of even finish, excellent coverage of uneven skin tone, the suppression of smudged makeup, the obtainment of glossy finish, and also excellent makeup durability. The mass ratio of the component (A) to the component (D), (A)/(D), is preferably from 0.3 to 15, more preferably from 0.5 to 7, further more preferably from 1 to 5.

In the present invention, the mass ratio of the component (B) to the component (D), (B)/(D), is preferably 0.01 or more, more preferably 0.03 or more, further more preferably 0.05 or more, and preferably 1 or less, more preferably 0.4 or less, further more preferably 0.2 or less, from the viewpoint of even finish, excellent coverage of uneven skin tone, the suppression of smudged makeup, the obtainment of glossy finish, and also excellent makeup durability. The mass ratio of the component (B) to the component (D), (B)/(D), is preferably from 0.01 to 1, more preferably from 0.03 to 0.4, further more preferably from 0.05 to 0.2.

In the present invention, the mass ratio of the component (C) to the component (D), (C)/(D), is preferably 0.2 or more, more preferably 0.4 or more, further more preferably 0.6 or more, and preferably 5 or less, more preferably 3 or less, further more preferably 1 or less, from the viewpoint of even finish, excellent coverage of uneven skin tone, the suppression of smudged makeup, the obtainment of glossy finish, and also excellent makeup durability. The mass ratio of the component (C) to the component (D), (C)/(D), is preferably from 0.2 to 5, more preferably from 0.4 to 3, further more preferably from 0.6 to 1.

The component (E) used in the present invention is an ester oil which is in a liquid state at 25°C.

The liquid state at 25°C refers to having a viscosity of 10 000 mPa·s or less at 25°C.

The viscosity is measured under the following conditions.

Viscosity measurement conditions: B type viscometer (TVB-10 model, manufactured by Toki Sangyo Co., Ltd.), rotor No. 4, 60 rpm, 60 sec.

The ester oil as the component (E) may not be limited as long as it can be used in usual cosmetics. Examples thereof include: monoester oils such as ethylhexyl p-methoxycinnamate, cetyl ethylhexanoate, octyldodecyl myristate, isotridecyl isononanoate, and isononyl isononanoate; diester oils such as neopentyl glycol dicaprate, propylene glycol dicaprate, and diisostearyl malate; triester oils such as glyceryl tri-2-ethylhexanoate and glyceryl tri(caprylate/caprate); and tetraester oils such as dipentaerythrityl tetraisostearate.

Among them, an ester oil having a molecular weight of 300 or larger is preferred from the viewpoint of excellent finish and the suppression of smudged makeup. The ester oil preferably comprises one or more members selected from the group consisting of monoester and diester, more preferably comprises one or more members selected from the group consisting of diisostearyl malate, neopentyl glycol dicaprate, isotridecyl isononanoate, cetyl ethylhexanoate, and propylene glycol dicaprate, and further more preferably comprise at least one or two members selected from the group consisting of diisostearyl malate and neopentyl glycol dicaprate.

One of or two or more in combination of these components (E) can be used. The content is preferably 3% by mass or more, more preferably 6% by mass or more, further more preferably 10% by mass or more, and preferably 40% by mass or less, more preferably 25% by mass or less, further more preferably 16% by mass or less, in the entire composition of the water-in-oil emulsion composition from the viewpoint of excellent finish and the suppression of smudged makeup. The content of the component (D) is preferably from 3 to 40% by mass, more preferably from 6 to 25% by mass, further more preferably from 10 to 16% by mass, in the entire composition of the water-in-oil emulsion composition.

The water-in-oil emulsion composition of the present invention can further comprise a volatile oil.

The term "volatile" refers to having a flash point of from -4 to 88°C. Examples of the volatile oil include volatile silicone oils and volatile hydrocarbon oils.

Examples of the volatile silicone oil include: linear dimethylpolysiloxane such as hexamethyldisiloxane, octamethyltrisiloxane (1 cs), decamethyltetrasiloxane (1.5 cs), and dimethylpolysiloxane (2 cs); branched siloxane such as methyl trimethicone, tris(trimethylsilyl)methylsilane, and tetrakis(trimethylsilyl)silane; and cyclic dimethylsiloxane such as octamethylcyclotetrasiloxane, decamethylcyclopentasiloxane, and dodecamethylcyclohexasiloxane.

Examples of the volatile hydrocarbon oil include: paraffin hydrocarbon oils such as n-decane, n-undecane, and n-dodecane; isoparaffin hydrocarbon oils such as isodecane, isododecane, and hydrogenated polyisobutene; and cyclic paraffin hydrocarbon oils such as cyclodecane and cyclododecane. Among them, a hydrocarbon oil having 8 to 16 carbon atoms is preferred, a hydrocarbon oil having 10 to 16 carbon atoms is more preferred, and a hydrocarbon oil having 12 carbon atoms is further more preferred.

The volatile oil is one or more members selected from the group consisting of a hydrocarbon oil and a silicone oil, and preferably comprises one or more members selected from the group consisting of isododecane, hexamethyldisiloxane, methyl trimethicone, and dimethylpolysiloxane having a kinetic viscosity of 2 cSt or less at 25°C, from the viewpoint of excellent finish and the suppression of smudged makeup. The kinetic viscosity can be measured using, for example, an

Ubbelohde viscometer.

Examples of commercially available products of the hexamethyldisiloxane and the dimethylpolysiloxane having a kinetic viscosity of 2 cSt or less at 25°C include "KF-96L-0.65 cs" (hexamethyldisiloxane), "TMF1.5", "KF-96L-1 cs" (octamethyltrisiloxane), "KF-96L-1.5 cs", and "KF-96L-2 cs" manufactured by Shin-Etsu Chemical Co., Ltd., "SH200C Fluid 1 cs" and "SH200C Fluid 1.5 cs" manufactured by DuPont Toray Specialty Materials K.K., "TSF451-0.65" manufactured by Momentive Performance Materials Japan LLC, "BELSIL DM0.65" manufactured by Wacker Asahikasei Silicone Co., Ltd., and "XIAMETER PMX-200 SILICONE FLUID 0.65 CS" manufactured by Dow Toray Co., Ltd.

One of or two or more in combination of these volatile oils can be used. The content is preferably 1% by mass or more, more preferably 4% by mass or more, further more preferably 8% by mass or more, and preferably 50% by mass or less, more preferably 35% by mass or less, further more preferably 25% by mass or less, in the entire composition of the water-in-oil emulsion composition from the viewpoint of excellent finish and the suppression of smudged makeup. The content of the volatile oil is preferably from 1 to 50% by mass, more preferably from 4 to 35% by mass, further more preferably from 8 to 25% by mass, in the entire composition of the water-in-oil emulsion composition.

The water-in-oil emulsion composition of the present invention can further comprise alkyl-modified silicone which is in a liquid state at 25°C.

The liquid state at 25°C refers to having a viscosity of 10 000 mPa·s or less at 25°C, as in the component (E).

The alkyl-modified silicone is silicone containing an alkyl group, and the alkyl group is preferably a linear or branched alkyl group having 8 to 24 carbon atoms, more preferably 8 to 20 carbon atoms, further more preferably 8 to 16 carbon atoms, even more preferably 8 to 12 carbon atoms. The polysiloxane may be substituted at a side chain, one end, or both ends by the alkyl group.

Examples of such alkyl-modified silicone include caprylyl methicone, cetyl dimethicone, alkyl (C26-28) dimethicone, stearyl dimethicone, behenoxy dimethicone, and (acrylates/stearyl acrylate/dimethicone methacrylate) copolymers. Among them, caprylyl methicone, cetyl dimethicone, alkyl (C26-28) dimethicone, stearyl dimethicone, or behenoxy dimethicone is preferred, and caprylyl methicone is more preferred.

One of or two or more in combination of these alkyl-modified silicones which are in a liquid state at 25°C can be used. The content is preferably 1% by mass or more, more preferably 2% by mass or more, further more preferably 3% by mass or more, and preferably 15% by mass or less, more preferably 10% by mass or less, further more preferably 8% by mass or less, in the entire composition of the water-in-oil emulsion composition from the viewpoint of even finish and excellent coverage of uneven skin tone. The content of the alkyl-modified silicone which is in a liquid state at 25°C is preferably from 1 to 15% by mass, more preferably from 2 to 10% by mass, further more preferably from 3 to 8% by mass, in the entire composition of the water-in-oil emulsion composition.

The water-in-oil emulsion composition of the present invention preferably has a content of an organic resin powder such as microplastics of 1% by mass or less, more preferably 0.1% by mass or less, in the entire composition, and further more preferably is substantially free from an organic resin powder, from the viewpoint of environmental friendliness, excellent use impression, and the obtainment of glossy finish.

Specifically, the organic resin powder refers to other than a cellulose powder excellent in biodegradability.

Examples of the organic resin powder include: polyamide resin, nylon resin, polyester resin, polyethylene resin, polyethylene tetrafluoride resin, polypropylene resin, polystyrene resin, benzoguanamine resin, polymethylbenzoguanamine resin, polyurethane resin, vinyl resin, fluorine resin, acrylic resin, and melamine resin; cross-linked or non-cross-linked organic resin powders such as powders of one or more polymers or copolymers selected from the group consisting of butyl acrylate-vinyl acetate copolymers, styrene-acrylic acid copolymers, silicone resin, and divinylbenzene-styrene copolymers.

In the water-in-oil emulsion composition of the present invention, the content of water is preferably from 3 to 50% by mass, more preferably from 5 to 40% by mass, further more preferably from 10 to 30% by mass, in the entire composition from the viewpoint of excellent use impression and the formation of a stable emulsion composition.

In the water-in-oil emulsion composition of the present invention, various components usually used in cosmetics can be optionally used, in addition to the components described above. Examples of these components include oil components other than those described above, surfactants, water-soluble polymers, antioxidants, ultraviolet absorbers, vitamins, antiseptics, pH adjusters, fragrances, plant extracts, humectants, colorants, cooling agents, antiperspirants, germicides, and skin activators.

The water-in-oil emulsion composition of the present invention can be produced in accordance with a usual method and can be prepared in a dosage form such as a liquid, emulsion, paste, cream, gel, or solid form.

The water-in-oil emulsion composition of the present invention can be prepared into a water-in-oil emulsion cosmetic and can be applied as, for example, a makeup cosmetic such as a makeup base, a foundation, a concealer, a blusher, an eye shadow, a mascara, an eyeliner, an eyebrow liner, an overcoat, or a lipstick; an ultraviolet protection cosmetic such as a sunscreen emulsion or a sunscreen cream; or a skincare cosmetic such as a skincare emulsion, a skincare cream, a BB cream, or a serum.

Among them, the water-in-oil emulsion composition of the present invention is suitable as a water-in-oil emulsion

cosmetic in a liquid state.

The viscosity of the water-in-oil emulsion composition of the present invention at 30°C is preferably from 3 000 to 30 000 mPa·s, more preferably from 5 000 to 25 000 mPa·s, from the viewpoint of even finish, excellent coverage of uneven skin tone, the suppression of smudged makeup, and the obtainment of glossy finish.

The viscosity is measured using a B type viscometer (manufactured by Toki Sangyo Co., Ltd., TVB-10 model) under conditions involving the number of rotations of 12 rpm, a measurement time of 30 seconds, and a temperature of 30°C. The rotors used are the following rotors depending on the viscosity of the composition.

Viscosity of 2 500 mPa·s or more and less than 10 000 mPa·s: rotor No. 3, and

Viscosity of 10 000 mPa·s or more: rotor No. 4

In Table 1, a water-in-oil emulsion composition of Example 1 had a viscosity of 12 000 mPa·s at 30°C.

The water-in-oil emulsion composition of the present invention can be used as a water-in-oil emulsion composition for impregnation by impregnating a holder with the composition and taking the composition by the finger or an applicator from the holder to apply the composition to the skin.

The holder is a material which holds the water-in-oil emulsion composition having flowability by impregnation. Examples thereof include nonwoven fabrics made of a single or mixed material such as resin, pulp, and cotton, resonated fiber materials, foams such as sponge, porous materials having a continuous mechanism, and assemblies of fibers.

Examples of the material include NBR (acrylonitrile-butadiene rubber), SBR (styrene-butadiene rubber), NR (natural rubber), urethane, nylon, polyolefin, polyester, EVA (ethylene vinyl acetate), PVA (polyvinyl alcohol), and silicone elastomers. Any material may be used as long as the holder can have contents. Among them, an assembly of polyester fibers is preferred. It is more preferred to use a fiber material formed using polyester fibers with a hardness of preferably from 40 to 60 (value measured with an F durometer) and an apparent density (which abides by JIS K 6400-1) of preferably from 0.006 to 0.1 g/cm$^3$, more preferably from 0.02 to 0.1 g/cm$^3$, which may be an assembly of polyester fibers having a layer structure where front and back layers are connected through cross-linked threads.

Among them, examples of the assembly of fibers include assemblies of fibers having a core-sheath structure. The fibers having a core-sheath structure are fibers having an internal core moiety surrounded by a sheath moiety. Preferably, the fibers are made of a thermoplastic resin, and the core moiety is constituted by a thermoplastic resin having a higher melting point than that of the sheath moiety. Materials for the core moiety and the sheath moiety in the fibers having a core-sheath structure are preferably polyolefin resin which is free from time-dependent deterioration ascribable to the components of the emulsion composition, though they are not particularly limited.

The polyolefin resin which can be used has the core moiety constituted by polypropylene (melting point: 165°C) and the sheath moiety constituted by polyethylene (melting point: 130°C), or has the core moiety constituted by polypropylene (melting point: 165°C) and the sheath moiety constituted by polybutene (melting point: 127°C). The fibers having a core-sheath structure preferably have a thickness of from 2.0 to 15 dtex, more preferably from 2.0 to 13 dtex, from the viewpoint of holding the emulsion composition by impregnation.

Eccentric core-sheath composite fibers are preferred for three-dimensional crimps. The shape of the core moiety in the eccentric core-sheath type may be a circle as well as a variant shape such as an oval, Y, or X shape, and the sectional shape of the eccentric core-sheath fibers may also be a circle as well as a variant shape such as an oval, Y, X, hash mark, polygonal, or star shape, or a hollow shape.

The assembly of the fibers having a core-sheath structure which can be used can be produced by a known method. Examples thereof include a thermal bond method, a needle punch method, and a spun lacing method. The assembly of the fibers having a core-sheath structure more preferably has partially fused sheath moieties in the fibers having a core-sheath structure. The assembly of the fibers having a core-sheath structure is not limited by its production method, and the method can be performed by, for example, a blast conveyor furnace scheme, a molding scheme, or a method using a mesh belt (method described in JP-B-4195043). Then, a size suitable for the holder can be created by cutting treatment such as pressing, though the method is not limited thereto.

The assembly of the fibers having a core-sheath structure more preferably has an apparent density (which abides by JIS K 6400-1) of from 0.02 to 0.1 g/cm$^3$ from the viewpoint of the holdability of the emulsion composition by impregnation.

The size of the assembly of the fibers having a core-sheath structure is not particularly limited and can be determined depending on, for example, easy use or the size of a container or the like in which it is to be housed.

The holder may be in a state wholly or partially impregnated with the water-in-oil emulsion composition or may be disposed as a screen net on the surface of the water-in-oil emulsion composition.

The weaving form of the screen net is not limited by its yarn texture configuration method, yarn shape, yarn twist count, thickness, material, or the like and may be, for example, plain weave, 1/2 twill weave, 2/1 twill weave, twill weave, leno texture, half-leno texture, or satin weave.

Examples of the screen net include screen nets made of polyurethane, TPE (thermoplastic plastic elastomer), polyester, polyether, acryl, or olefin. The thickness of the fibers constituting the screen net is preferably from 0.01 to 1.0 mm.

It is preferred that the screen net should be completely attached to the holder impregnated with the emulsion composition by tight contact, or an absorber should be positioned 0.1 to 3.0 mm distant from the screen net without adhesion between the absorber and the screen net.
The water-in-oil emulsion composition of the present invention can be used by impregnating such a holder therewith and taking the composition by the finger or an applicator from the holder to apply the composition to the skin.
The water-in-oil emulsion composition of the present invention can be used by impregnating such a holder therewith, positioning a screen net adjacently therewith, and taking the composition by the finger or an applicator from the screen net to apply the composition to the skin.
Examples of the applicator for application to the skin include sponge, puff, and chips which are usually used in applying liquid cosmetics to the skin.
The water-in-oil emulsion composition of the present invention can be prepared into a cosmetic in which a holder impregnated with the water-in-oil emulsion composition is housed in a compact container.
The water-in-oil emulsion composition of the present invention can be prepared into a cosmetic in which a holder impregnated with the water-in-oil emulsion composition and a screen net are positioned adjacently with each other and are housed in a compact container.
The water-in-oil emulsion composition of the present invention can be prepared into a cosmetic kit comprising a set of a holder impregnated with the water-in-oil emulsion composition, and a compact container.
The water-in-oil emulsion composition of the present invention can be used by the impregnation of a holder and, in addition, may be suitably used as a usual liquid water-in-oil emulsion cosmetic, particularly, a liquid foundation, without impregnating the holder therewith.
In relation to the embodiments mentioned above, the present invention further discloses the following compositions and the like.

<1> A water-in-oil emulsion composition for impregnation comprising the following components (A), (B), (C), (D), and (E) :

(A) a hydrophobized color pigment,
(B) an acrylic silicone polymer,
(C) one or more members selected from the group consisting of polyether-modified silicone and polyglyceryl ether-modified silicone,
(D) spherical silica, and
(E) an ester oil which is in a liquid state at 25°C.

<2> A cosmetic in which a holder impregnated with a water-in-oil emulsion composition is housed in a compact container, the water-in-oil emulsion composition comprising the following components (A), (B), (C), (D), and (E):

(A) a hydrophobized color pigment,
(B) an acrylic silicone polymer,
(C) one or more members selected from the group consisting of polyether-modified silicone and polyglyceryl ether-modified silicone,
(D) spherical silica, and
(E) an ester oil which is in a liquid state at 25°C.

<3> The cosmetic according to <2>, wherein the color pigment as the component (A) preferably comprises metal oxide, more preferably comprises one or more members selected from the group consisting of titanium oxide, zinc oxide, yellow iron oxide, black iron oxide, and red iron oxide, and further more preferably comprises one or more members selected from the group consisting of titanium oxide, yellow iron oxide, black iron oxide, and red iron oxide.
<4> The cosmetic according to <2> or <3>, wherein the hydrophobization treatment of the component (A) preferably comprises one or more members selected from the group consisting of amino acid treatment, acylated amino acid treatment, and alkoxysilane treatment, more preferably comprises one or more members selected from the group consisting of alkylalkoxysilane treatment, amino acid treatment, and acylated amino acid treatment, further more preferably comprises treatment with one or more members selected from the group consisting of alkylalkoxysilane, disodium stearoyl glutamate, sodium lauroyl aspartate, and isopropyl triisostearoyl titanate, even more preferably comprises treatment with one or more members selected from the group consisting of alkylalkoxysilane, disodium stearoyl glutamate, and isopropyl triisostearoyl titanate, and even more preferably comprises disodium stearoyl glutamate treatment.
<5> The cosmetic according to any one of <2> to <4>, wherein a content of the component (A) is preferably 1% by mass or more, more preferably 3% by mass or more, further more preferably 5% by mass or more, and preferably 30%

by mass or less, more preferably 25% by mass or less, further more preferably 20% by mass or less, in the entire composition of the water-in-oil emulsion composition.

<6> The cosmetic according to any one of <2> to <5>, wherein the component (B) is preferably a vinyl polymer having a carbosiloxane dendrimer structure in a side chain or an acryl-silicone graft copolymer, more preferably an acryl-silicone graft copolymer.

<7> The cosmetic according to <6>, wherein the vinyl polymer having a carbosiloxane dendrimer structure in a side chain is preferably a silicone dendrimer-acryl copolymer, more preferably an (acrylates/polytrimethylsiloxy methacrylate) copolymer.

<8> The cosmetic according to <6>, wherein the acryl-silicone graft copolymer is preferably an (acrylates/dimethicone) copolymer or an (acrylates/ethylhexyl acrylate/dimethicone methacrylate) copolymer, more preferably an (acrylates/ethylhexyl acrylate/dimethicone methacrylate) copolymer.

<9> The cosmetic according to any one of <2> to <8>, wherein a content of the component (B) is preferably 0.01% by mass or more, more preferably 0.1% by mass or more, further more preferably 0.3% by mass or more, and preferably 5% by mass or less, more preferably 3% by mass or less, further more preferably 1% by mass or less, in the entire composition of the water-in-oil emulsion composition.

<10> The cosmetic according to any one of <2> to <9>, wherein the component (C) is polyether-modified silicone, more preferably PEG-8 dimethicone, PEG-10 dimethicone, PEG-12 dimethicone, or PEG-14 dimethicone.

<11> The cosmetic according to any one of <2> to <9>, wherein the component (C) is polyglyceryl ether-modified silicone, preferably lauryl PEG-10 tris(trimethylsiloxy)silylethyl dimethicone, cetyl diglyceryl tris(trimethylsiloxy)silylethyl dimethicone, cetyl PEG/PPG-10/1 dimethicone, lauryl PEG-9 polydimethylsiloxyethyl dimethicone, or lauryl PEG/PPG-18/18 methicone.

<12> The cosmetic according to any one of <2> to <9>, wherein the component (C) is preferably alkyl glyceryl ether-modified silicone of the following formula (5):

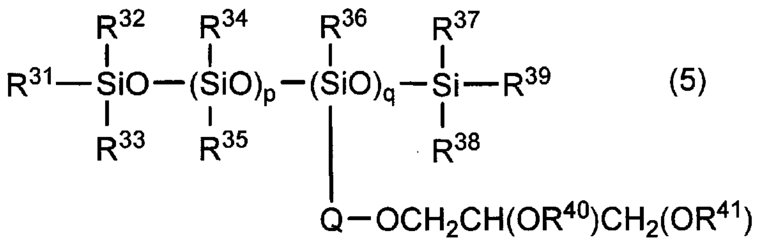

(5)

wherein $R^{31}$ and $R^{39}$ are the same or different and are each independently a linear or branched hydrocarbon group having 1 to 32 carbon atoms in terms of a mode value; $R^{32}$ to $R^{38}$ are the same or different and are each independently a linear or branched hydrocarbon group having 1 to 5 carbon atoms; Q is a linear or branched divalent hydrocarbon group having 3 to 20 carbon atoms; $R^{40}$ and $R^{41}$ are each independently a hydrogen atom or a linear or branched hydrocarbon group having 1 to 28 carbon atoms, and at least one of the moieties is a hydrogen atom; p represents the number of repeat units and is a number of 5 or more and 60 or less as an average value; q represents the number of repeat units and is a number of 2.5 or more and 10 or less as an average value; and constituent units with the number of repeats, p and q, are any of block copolymers and random copolymers, provided that the formula has one or more alkyl groups having 10 or more carbon atoms,

more preferably glyceryl undecyl dimethicone or bisalkyl (C16-18) glyceryl undecyl dimethicone.

<13> The cosmetic according to any one of <2> to <12>, wherein a content of the component (C) is preferably 0.1% by mass or more, more preferably 1% by mass or more, further more preferably 2% by mass or more, and preferably 12% by mass or less, more preferably 7% by mass or less, further more preferably 5% by mass or less, in the entire composition of the water-in-oil emulsion composition.

<14> The cosmetic according to any one of <2> to <13>, wherein the spherical silica as the component (D) preferably has a specific surface area of 150 $m^2/g$ or less, more preferably 60 $m^2/g$ or less, further more preferably 10 $m^2/g$ or less, and is even more preferably nonporous.

<15> The cosmetic according to any one of <2> to <14>, wherein the spherical silica as the component (D) preferably has a volume-average particle size of from 1 to 50 $\mu m$, more preferably from 1 to 35 $\mu m$, further more preferably from 2 to 25 $\mu m$, even more preferably from 3 to 15 $\mu m$.

<16> The cosmetic according to any one of <2> to <15>, wherein a content of the component (D) is preferably 0.1% by mass or more, more preferably 1% by mass or more, further more preferably 2% by mass or more, and preferably 20% by mass or less, more preferably 12% by mass or less, further more preferably 8% by mass or less, in the entire composition of the water-in-oil emulsion composition.

<17> The cosmetic according to any one of <2> to <16>, wherein a mass ratio of the component (A) to the component (D), (A)/(D), is preferably 0.3 or more, more preferably 0.5 or more, further more preferably 1 or more, and preferably 15 or less, more preferably 7 or less, further more preferably 5 or less.

<18> The cosmetic according to any one of <2> to <17>, wherein a mass ratio of the component (B) to the component (D), (B)/(D), is preferably 0.01 or more, more preferably 0.03 or more, further more preferably 0.05 or more, and preferably 1 or less, more preferably 0.4 or less, further more preferably 0.2 or less.

<19> The cosmetic according to any one of <2> to <18>, wherein a mass ratio of the component (C) to the component (D), (C)/(D), is preferably 0.2 or more, more preferably 0.4 or more, further more preferably 0.6 or more, and preferably 5 or less, more preferably 3 or less, further more preferably 1 or less.

<20> The cosmetic according to any one of <2> to <19>, wherein the component (E) is preferably an ester oil having a molecular weight of 300 or larger, more preferably comprises one or more members selected from the group consisting of monoester and diester, further more preferably comprises one or more members selected from the group consisting of diisostearyl malate, neopentyl glycol dicaprate, isotridecyl isononanoate, cetyl ethylhexanoate, and propylene glycol dicaprate, and even more preferably comprises at least one or two members selected from the group consisting of diisostearyl malate and neopentyl glycol dicaprate.

<21> The cosmetic according to any one of <2> to <20>, wherein a content of the component (E) is preferably 3% by mass or more, more preferably 6% by mass or more, further more preferably 10% by mass or more, and preferably 40% by mass or less, more preferably 25% by mass or less, further more preferably 16% by mass or less, in the entire composition of the water-in-oil emulsion composition.

<22> The cosmetic according to any one of <2> to <21>, wherein preferably, a content of the component (A) is from 1 to 30% by mass, a content of the component (B) is from 0.01 to 5% by mass, a content of the component (C) is from 0.1 to 12% by mass, a content of the component (D) is from 0.1 to 20% by mass, and a content of the component (E) is from 3 to 40% by mass.

<23> The cosmetic according to any one of <2> to <22>, wherein the water-in-oil emulsion composition preferably further comprises a volatile oil, and more preferably comprises 1 to 50% by mass of the volatile oil in the entire composition of the water-in-oil emulsion composition.

<24> The cosmetic according to <23>, wherein the volatile oil is preferably one or more members selected from the group consisting of a volatile silicone oil and a volatile hydrocarbon oil, and more preferably comprises one or more members selected from the group consisting of isododecane, hexamethyldisiloxane, methyl trimethicone, and dimethylpolysiloxane having a kinetic viscosity of 2 cSt or less at 25°C.

<25> The cosmetic according to <23> or <24>, wherein a content of the volatile oil is preferably 1% by mass or more, more preferably 4% by mass or more, further more preferably 8% by mass or more, and preferably 50% by mass or less, more preferably 35% by mass or less, further more preferably 25% by mass or less, in the entire composition of the water-in-oil emulsion composition.

<26> The cosmetic according to any one of <2> to <25>, wherein the water-in-oil emulsion composition preferably further comprises alkyl-modified silicone which is in a liquid state at 25°C, more preferably caprylyl methicone, cetyl dimethicone, alkyl (C26-28) dimethicone, stearyl dimethicone, or behenoxy dimethicone, further more preferably caprylyl methicone.

<27> The cosmetic according to <26>, wherein a content of the alkyl-modified silicone which is in a liquid state at 25°C is preferably 1% by mass or more, more preferably 2% by mass or more, further more preferably 3% by mass or more, and preferably 15% by mass or less, more preferably 10% by mass or less, further more preferably 8% by mass or less, in the entire composition of the water-in-oil emulsion composition.

<28> The cosmetic according to any one of <2> to <27>, wherein the water-in-oil emulsion composition preferably has an organic resin powder content of 1% by mass or less, more preferably 0.1% by mass or less, in the entire composition, and further more preferably is substantially free from an organic resin powder.

<29> The cosmetic according to any one of <2> to <28>, wherein a content of water in the water-in-oil emulsion composition is preferably from 3 to 50% by mass, more preferably from 5 to 40% by mass, further more preferably from 10 to 30% by mass, in the entire composition.

<30> The cosmetic according to any one of <2> to <29>, wherein the water-in-oil emulsion composition is preferably a water-in-oil emulsion cosmetic in a liquid state.

<31> The cosmetic according to any one of <2> to <30>, wherein a viscosity at 30°C of the water-in-oil emulsion composition is preferably from 3 000 to 30 000 mPa·s, more preferably from 5 000 to 25 000 mPa·s.

<32> The cosmetic according to any one of <2> to <31>, wherein the holder is preferably a foam, a porous material, or an assembly of fibers, more preferably an assembly of polyester fibers.

<33> A method for using the cosmetic according to any one of <2> to <32>, comprising taking a water-in-oil emulsion composition impregnated into a holder, by finger or an applicator from the holder, and applying it to the skin for use.

<34> A cosmetic kit comprising a set of a holder impregnated with a water-in-oil emulsion composition, and a compact container, the water-in-oil emulsion composition comprising the following components (A), (B), (C), (D), and (E):

(A) a hydrophobized color pigment,
(B) an acrylic silicone polymer,
(C) one or more members selected from the group consisting of polyether-modified silicone and polyglyceryl ether-modified silicone,
(D) spherical silica, and
(E) an ester oil which is in a liquid state at 25°C.

<35> A water-in-oil emulsion composition comprising the following components (A), (B), (C), (D), and (E):

(A) a hydrophobized color pigment,
(B) an acrylic silicone polymer,
(C) one or more members selected from the group consisting of polyether-modified silicone and polyglyceryl ether-modified silicone,
(D) spherical silica, and
(E) an ester oil which is in a liquid state at 25°C.

Examples

Production Example 1 (Production of both terminally alkyl (C16-18)-modified dimethylsiloxane-methyl(undecyl glyceryl ether)siloxane copolymer):

(1) Step-1 (Synthesis of tetramethyldisiloxane having, at both ends of silicone chain, alkyl groups different from other alkyl groups in the silicone chain):

44.8 g of 1,1,3,3-tetramethyldisiloxane and 1.0 g of Spier's catalyst (solution containing 2% by mass of chloroplatinic acid in 2-propanol) were added to a three-neck flask and warmed to 70°C. In a nitrogen atmosphere, 174.2 g of $\alpha$-olefin (manufactured by Idemitsu Kosan Co., Ltd., "LINEAREN 168", a 57/43 (mass ratio) mixture of olefins having 16 and 18 carbon atoms)) was added dropwise thereto at 70°C, followed by stirring for 2 hours. After cooling, the reaction system was neutralized with an aqueous sodium hydroxide solution, and distillation and purification were performed under reduced pressure. The obtained product was confirmed from the $^1$H-NMR spectrum (400 MHz) of the obtained product to be a 1,1,3,3-tetramethyldisiloxane derivative having alkyl groups having 16 carbon atoms and having 18 carbon atoms at both ends (22.1 g, yield; 85%).

(2) Step-2 (Synthesis of organo hydrogen polysiloxane having, at both ends of silicone chain, alkyl groups different from other alkyl groups in the silicone chain, and having silicon-hydrogen bond in the silicone chain):

44.8 g of the 1,1,3,3-tetramethyldisiloxane derivative having alkyl groups having 16 carbon atoms and having 18 carbon atoms at both ends synthesized in (1), 78.6 g of decamethylcyclopentasiloxane, 19.8 g of 1,3,5,7-tetramethylcyclotetrasiloxane, 50 g of n-heptane, and 5 g of activated earth were added to a three-neck flask and refluxed for 12 hours. After cooling, distillation and purification were performed under reduced pressure. The obtained product was confirmed from the $^1$H-NMR spectrum of the obtained product to be a dimethylsiloxane/methylsiloxane copolymer (p = 23, q = 4) having alkyl groups having 16 carbon atoms and having 18 carbon atoms at both ends (132.8 g, yield; 95%).

(3) Step-3 (Synthesis of polysiloxane both terminally substituted by alkyl groups and modified at side chain with alkyl glyceryl ether group in graft pattern):

50.0 g of the dimethylsiloxane/methylsiloxane copolymer having alkyl groups having 16 carbon atoms and having 18 carbon atoms at both ends synthesized in (2), 61.0 g of 10-undecenyl glyceryl ether, and 0.25 g of 5% by mass of platinum catalyst supported on carbon were added to a three-neck flask and stirred at 70°C for 3 hours. After cooling, distillation and purification were performed under reduced pressure. The obtained product was confirmed from the $^1$H-NMR spectrum of the obtained product to be a dimethylsiloxane-methyl(undecyl glyceryl ether)siloxane copolymer (p = 23, q = 4) having alkyl groups having 16 carbon atoms and having 18 carbon atoms at both ends (63.0 g, yield; 95%).
The obtained copolymer is a random copolymer of the formula (5) as given below.

$R^{31}$ and $R^{39}$ = an alkyl group having 18 carbon atoms in terms of a mode value,
$R^{32}$ to $R^{38}$ = a methyl group,
Q = an alkylene group having 11 carbon atoms,
$R^{40}$ and $R^{41}$ = a hydrogen atom

p (average value) = 23,
q (average value) = 4.0

Examples 1 to 7 and Comparative Examples 1 and 2

Water-in-oil emulsion cosmetics (liquid foundations) were produced in accordance with the composition shown in Table 1.

The obtained water-in-oil emulsion cosmetics were evaluated for their evenness of finish, coverage of uneven skin tone, glossy finish, and unlikeliness to cause smudged makeup, after a holder was impregnated with each cosmetic. The results are also shown in Table 1.

(Production method)

A powder component comprising the components (A) and (D) was added to and dispersed in an oil phase component comprising the components (B), (C), and (E). Then, a water phase component was added thereto and stirred with a homomixer to produce a water-in-oil emulsion cosmetic (liquid foundation).

A holder (assembly of polyester fibers) was impregnated with the obtained water-in-oil emulsion cosmetic.

(Evaluation method)

(1) Evenness of finish:

Three expert panelists used puff to apply each water-in-oil emulsion composition (foundation with the holder impregnated therewith) to the skin, and conducted sensory evaluation on the evenness of finish in accordance with criteria given below. The results were indicated by the total score of the three panelists.

5; Uneven application was not noticeable at all.
4; Uneven application was not noticeable.
3; Uneven application was rarely noticeable.
2; Uneven application was slightly noticeable.
1; Uneven application was noticeable.

(2) Coverage of uneven skin tone:

Three expert panelists used puff to apply each water-in-oil emulsion composition (foundation with the holder impregnated therewith) to the skin, and conducted sensory evaluation on the coverage of uneven skin tone in accordance with criteria given below. The results were indicated by the total score of the three panelists.

5; Uneven skin tone was not noticeable at all.
4; Uneven skin tone was not noticeable.
3; Uneven skin tone was rarely noticeable.
2; Uneven skin tone was slightly noticeable.
1; Uneven skin tone was noticeable.

(3) Glossy finish:

Three expert panelists used puff to apply each water-in-oil emulsion composition (foundation with the holder impregnated therewith) to the skin, and conducted sensory evaluation on the gloss of finish in accordance with criteria given below. The results were indicated by the total score of the three panelists.

5; Looked very glossy
4; Looked glossy
3; Looked slightly glossy
2; Rarely looked glossy
1; Not looked glossy

(4) Unlikeliness to cause smudged makeup:

Three expert panelists used puff to apply each water-in-oil emulsion composition (foundation with the holder impregnated therewith) to the skin, and immediately thereafter, conducted sensory evaluation on smudged makeup in accordance with criteria given below. The results were indicated by the total score of the three panelists.

5; Smudged makeup was totally absent.
4; Smudged makeup was absent.
3; Smudged makeup was rarely present.
2; Smudged makeup was slightly present.
1; Smudged makeup was present.

[Table 1]

| | | Component name | Starting material name | Example 1 | Example 2 | Example 3 | Example 4 | Example 5 | Example 6 | Example 7 | Comparative Example 1 | Comparative Example 2 |
|---|---|---|---|---|---|---|---|---|---|---|---|---|
| Oil phase | (B) | (Acrylates/ethylhexyl acrylate/dimethicone methacrylate) copolymer | manufactured by Shin-Etsu Chemical Co., Ltd., KP-578 | 0.50 | 0.40 | 0.70 | 0.50 | 0.50 | 0.50 | 0.50 | 0.50 | - |
| | (C) | Dimethylsiloxane-methyl(undecyl glyceryl ether)siloxane copolymer | | 1.00 | 1.00 | 1.00 | 0.50 | 1.50 | 1.00 | 1.00 | 1.00 | 1.00 |
| | | Both terminally alkyl (C16-18)-modified dimethylsiloxane-methyl(undecyl glyceryl ether)siloxane copolymer | Production Example 1 | 0.50 | 0.50 | 0.50 | 0.25 | 0.75 | 0.50 | 0.50 | 0.50 | 0.50 |
| | | Methylpolysiloxane-cetylmethylpolysiloxane-poly(oxyethylene-oxypropylene)methylpolysiloxane copolymer | manufactured by Evonik Industries AG, ABIL EM 90 | 2.50 | 2.50 | 2.50 | 2.50 | 2.50 | 2.50 | 2.50 | 2.50 | 2.50 |
| | Other components | Methylpolysiloxane | manufactured bv Shin-Etsu Chemical Co., Ltd., Silicone KF-96L-2CS | 14.00 | 14.00 | 14.00 | 14.00 | 14.00 | 14.00 | 14.00 | 14.00 | 14.50 |
| | | Methylphenylpolysiloxane-dimethyl distearyl ammonium hectorite-triethyl citrate mixture | manufactured by Elementis Specialties, Inc., BENTONE GEL PTM V | 10.00 | 10.00 | 10.00 | 10.00 | 10.00 | 10.00 | 10.00 | 10.00 | 10.00 |
| | | Caprylyl methicone | manufactured by Momentive Performance Materials, Inc., SILSOFT 034 | 5.00 | 5.00 | 5.00 | 5.00 | 5.00 | 5.00 | 5.00 | 5.00 | 5.00 |

(continued)

| | | Component name | Starting material name | Example 1 | Example 2 | Example 3 | Example 4 | Example 5 | Example 6 | Example 7 | Comparative Example 1 | Comparative Example 2 |
|---|---|---|---|---|---|---|---|---|---|---|---|---|
| Water phase | (E) | Diisostearyl malate | manufactured by Nisshin OilliO Group, Ltd., COSMOL 222 | 7.00 | 7.00 | 7.00 | 7.00 | 7.00 | 7.00 | 7.00 | 7.00 | 7.00 |
| | | Neopentyl glycol dicaprate | manufactured by Nisshin OilliO Group, Ltd., ESTEMOL N-01 | 5.00 | 5.10 | 4.80 | 5.75 | 4.25 | 5.00 | 5.00 | 5.00 | 5.00 |
| | Other compone nts | Dipropylene glycol | | 4.00 | 4.00 | 4.00 | 4.00 | 4.00 | 4.00 | 4.00 | 4.00 | 4.00 |
| | | 1,3-Butylene glycol | | 6.00 | 6.00 | 6.00 | 6.00 | 6.00 | 6.00 | 6.00 | 6.00 | 6.00 |
| | | Diglycerin | | 1.00 | 1.00 | 1.00 | 1.00 | 1.00 | 1.00 | 1.00 | 1.00 | 1.00 |
| | | Sodium chloride | | 0.50 | 0.50 | 0.50 | 0.50 | 0.50 | 0.50 | 0.50 | 0.50 | 0.50 |
| | | Methyl p-hydroxybenzoate | | 0.20 | 0.20 | 0.20 | 0.20 | 0.20 | 0.20 | 0.20 | 0.20 | 0.20 |
| | | Phenoxyethanol | | 0.50 | 0.50 | 0.50 | 0.50 | 0.50 | 0.50 | 0.50 | 0.50 | 0.50 |
| | | Water-soluble collagen solution (3) | manufactured by Ichimaru Pharcos Co., Ltd., NEPTIGEN N | 0.10 | 0.10 | 0.10 | 0.10 | 0.10 | 0.10 | 0.10 | 0.10 | 0.10 |
| | | Sodium hyaluronate(2) | manufactured by Kikkoman Biochemifa Company, Hyaluronic Acid FCH(FCH-SU) | 0.01 | 0.01 | 0.01 | 0.01 | 0.01 | 0.01 | 0.01 | 0.01 | 0.01 |
| | | BHT | | 0.02 | 0.02 | 0.02 | 0.02 | 0.02 | 0.02 | 0.02 | 0.02 | 0.02 |
| | | Purified water | | 18.927 | 18.927 | 18.927 | 18.927 | 18.927 | 20.827 | 16.927 | 18.927 | 18.927 |
| Powder phase | (A) | n-Octylsilylated titanium oxide | manufactured by Daito Kasei Koavo Co., Ltd., OTS-2 MP-40 | 5.00 | 5.00 | 5.00 | 5.00 | 5.00 | 5.00 | 5.00 | 5.00 | 5.00 |

(continued)

| | | Component name | Starting material name | Example 1 | Example 2 | Example 3 | Example 4 | Example 5 | Example 6 | Example 7 | Comparative Example 1 | Comparative Example 2 |
|---|---|---|---|---|---|---|---|---|---|---|---|---|
| | | n-Octylsilylated Blue No. 1 aluminum lake | manufactured by Daito Kasei Kogyo Co., Ltd., OTS-5 BC BLUE NO.1 AL LAKE | 0.003 | 0.003 | 0.003 | 0.003 | 0.003 | 0.003 | 0.003 | 0.003 | 0.003 |
| | | Isopropyl triisostearoyl titanate-modified Red No. 202 | manufactured by Daito Kasei Koavo Co., Ltd., ITT-10 BC Red No. 202 K | 0.04 | 0.04 | 0.04 | 0.04 | 0.04 | 0.04 | 0.04 | 0.04 | 0.04 |
| | | Dimethicone-disodium stearoyl glutamate composite-treated titanium oxide | manufactured by Miyoshi Kasei, Inc., SA/-NAI-TR-10 MIBRID COLOR POWDER | 8.00 | 8.00 | 8.00 | 8.00 | 8.00 | 8.00 | 8.00 | 8.00 | 8.00 |
| | | Dimethicone-disodium stearoyl glutamate composite-treated red iron oxide | manufactured by Miyoshi Kasei, Inc., SA/-NAI-R-10 MIBRID COLOR POWDER | 0.34 | 0.34 | 0.34 | 0.34 | 0.34 | 0.34 | 0.34 | 0.34 | 0.34 |
| | | Dimethicone-disodium stearoyl glutamate composite-treated black iron oxide | manufactured by Miyoshi Kasei, Inc., SA/-NAI-B-10 MIBRID COLOR POWDER | 0.06 | 0.06 | 0.06 | 0.06 | 0.06 | 0.06 | 0.06 | 0.06 | 0.06 |
| | | Dimethicone-disodium stearoyl glutamate composite-treated yellow iron oxide | manufactured by Miyoshi Kasei, Inc., SA/-NAI-Y-10 MIBRID COLOR POWDER | 1.00 | 1.00 | 1.00 | 1.00 | 1.00 | 1.00 | 1.00 | 1.00 | 1.00 |

(continued)

| | | Component name | Starting material name | Example 1 | Example 2 | Example 3 | Example 4 | Example 5 | Example 6 | Example 7 | Comparative Example 1 | Comparative Example 2 |
|---|---|---|---|---|---|---|---|---|---|---|---|---|
| | (D) | Silicic anhydride | manufactured by Momentive Performance Materials Japan LLC, HARMONIE LUXE 4 POWDER (specific surface area: 1.3 $m^2/g$) | 5.00 | 5.00 | 5.00 | 5.00 | 5.00 | 3.10 | 7.00 | - | 5.00 |
| | Other components | Chromium hydroxide | manufactured by Sensient Cosmetic Technologies SAS, UNIPURE GREEN LC 790 CF | 0.10 | 0.10 | 0.10 | 0.10 | 0.10 | 0.10 | 0.10 | 0.10 | 0.10 |
| | | Amino acid-isopropyl triisostearoyl titanate composite-treated titanated mica | Amino acid-isopropyl triisostearoyl titanate composite-treated, manufactured by Merck KGaA, LUMINOUS GLOW | 3.70 | 3.70 | 3.70 | 3.70 | 3.70 | 3.70 | 3.70 | 3.70 | 3.70 |
| | | Polymethylsilsesquioxane | manufactured by Momentive Performance Materials Japan LLC, TOSPEARL 145A | | | | | | | | 5.00 | |
| Total | | | | 100.00 | 100.00 | 100.00 | 100.00 | 100.00 | 100.00 | 100.00 | 100.00 | 100.00 |
| Total (A) | | | | 14.44 | 14.44 | 14.44 | 14.44 | 14.44 | 14.44 | 14.44 | 14.44 | 14.44 |
| Total (B) | | | | 0.50 | 0.40 | 0.70 | 0.50 | 0.50 | 0.50 | 0.50 | 0.50 | |

(continued)

| | Component name | Starting material name | Example 1 | Example 2 | Example 3 | Example 4 | Example 5 | Example 6 | Example 7 | Comparative Example 1 | Comparative Example 2 |
|---|---|---|---|---|---|---|---|---|---|---|---|
| Total (C) | | | 4.00 | 4.00 | 4.00 | 3.25 | 4.75 | 4.00 | 4.00 | 4.00 | 4.00 |
| Total (D) | | | 5.00 | 5.00 | 5.00 | 5.00 | 5.00 | 3.10 | 7.00 | - | 5.00 |
| Total (E) | | | 12.00 | 12.10 | 11.80 | 12.75 | 11.25 | 12.00 | 12.00 | 12.00 | 12.00 |
| (A)/(D) | | | 2.89 | 2.89 | 2.89 | 2.89 | 2.89 | 4.66 | 2.06 | - | - |
| (B)/(D) | | | 0.10 | 0.08 | 0.14 | 0.10 | 0.10 | 0.16 | 0.07 | - | - |
| (C)/(D) | | | 0.80 | 0.80 | 0.80 | 0.65 | 0.95 | 1.29 | 0.57 | - | - |
| Evaluation | Evenness of finish | | 15 | 14 | 15 | 15 | 14 | 14 | 14 | 11 | 8 |
| | coverage of uneven skin tone | | 15 | 14 | 15 | 14 | 14 | 14 | 13 | 11 | 9 |
| | Glossy finish | | 15 | 14 | 15 | 14 | 13 | 15 | 13 | 9 | 10 |
| | Unlikeliness to cause smudged makeup | | 15 | 15 | 15 | 13 | 14 | 13 | 14 | 14 | 8 |

The water-in-oil emulsion cosmetics (liquid foundations) of Examples 1 to 7 can be used by filling each cosmetic into a container such as a bottle container or a tube container, without impregnation of a holder, and taking it by the finger or an applicator directly from the container to apply the cosmetic to the skin. In this case as well, fit feeling to the skin is excellent, and emulsification stability is also excellent.

Examples 8 and 9

Water-in-oil emulsion cosmetics (liquid foundations) were produced in accordance with the composition shown in Table 2 in the same manner as in Examples 1 to 7, and a holder (assembly of polyester fibers) was impregnated with each of the obtained water-in-oil emulsion cosmetics.
The obtained cosmetics offer even finish, are excellent in coverage of uneven skin tone, are unlikely to cause smudged makeup, produce glossy finish, and are also excellent in makeup durability.
The obtained water-in-oil emulsion cosmetics can also be used by filling each cosmetic into a container such as a bottle container or a tube container, without impregnation of a holder, and taking it by the finger or an applicator directly from the container to apply the cosmetic to the skin. In this case as well, fit feeling to the skin is excellent, and emulsification stability is also excellent.

[Table 2]

| | | Component name | Starting material name | Example 8 | Example 9 |
|---|---|---|---|---|---|
| Oil phase | (B) | (Acrylates/ethylhexyl acrylate/dimethicone methacrylate) copolymer | manufactured by Shin-Etsu Chemical Co., Ltd., KP-578 | 0.50 | 0.50 |
| | (C) | Dimethylsiloxane-methyl(undecyl glyceryl ether)siloxane copolymer | | 1.00 | 1.00 |
| | | Both terminally alkyl (C16-18)-modified dimethylsiloxane-methylfundecyl glyceryl ether)siloxane copolymer | Production Example 1 | 0.50 | 0.50 |
| | | Methylpolysiloxane-cetylmethylpolysiloxane-poly(oxvethylene-oxvpropylene)methylpolysiloxane copolymer | manufactured by Evonik Industries AG, ABIL EM 90 | 2.50 | 2.50 |
| | Other components | Methylpolysiloxane | manufactured by Shin-Etsu Chemical Co., Ltd., Silicone KF-96L-2CS | 14.00 | 14.00 |
| | | Methylphenylpolysiloxane-dimethyl distearyl ammonium hectorite-triethyl citrate mixture | manufactured by Elementis Specialties, Inc., BENTONE GEL PTM V | 10.00 | 10.00 |
| | | Caprylyl methicone Caprylyl methicone | manufactured by Momentive Performance Materials, Inc., SILSOFT 034 | 5.00 5.00 | 5.00 5.00 |
| | (E) | Diisostearyl malate | manufactured by Nisshin OilliO Group, Ltd., COSMOL 222 | 7.00 | 7.00 |
| | | Neopentyl alvcol dicaprate | manufactured by Nisshin OilliO Group, Ltd., ESTEMOL N-01 | 5.00 | 5.10 |

(continued)

| | | Component name | Starting material name | Example 8 | Example 9 |
|---|---|---|---|---|---|
| Water phase | Other components | Dipropylene glycol | | 4.00 | 4.00 |
| | | 1,3-Butylene glycol | | 6.00 | 6.00 |
| | | Diglycerin | | 1.00 | 1.00 |
| | | Sodium chloride | | 0.50 | 0.50 |
| | | Methyl p-hydroxybenzoate | | 0.20 | 0.20 |
| | | Phenoxvethanol | | 0.50 | 0.50 |
| | | Water-soluble collaaen solution (3) | manufactured by Ichimaru Pharcos Co., Ltd., NEPTIGEN N | 0.10 | 0.10 |
| | | Sodium hyaluronate (2) | manufactured by Kikkoman Biochemifa Company, Hyaluronic Acid FCH(FCH-SU) | 0.01 | 0.01 |
| | | BHT | | 0.02 | 0.02 |
| | | Purified water | | 18.927 | 17.887 |
| Powder phase | (A) | n-Octylsilylated titanium oxide | manufactured by Daito Kasei Koavo Co., Ltd., OTS-2 MP-40 | 5.00 | 5.00 |
| | | n-Octylsilylated Blue No. 1 aluminum lake | manufactured by Daito Kasei Koavo Co., Ltd., OTS-5 BC BLUE NO.1 AL LAKE | 0.003 | 0.003 |
| | | Isopropyl triisostearoyl titanate-modified Red No. 202 | manufactured by Daito Kasei Koavo Co., Ltd., ITT-10 BC Red No. 202 K | 004 | 004 |
| | | Dimethicone-disodium stearoyl glutamate composite-treated titanium oxide | manufactured by Miyoshi Kasei, Inc., SA/NAI-TR-10 MIBRID COLOR POWDER | 8.00 | |
| | | Dimethicone-disodium stearoyl glutamate composite-treated red iron oxide | manufactured by Miyoshi Kasei, Inc., SA/NAI-R-10 MIBRID COLOR POWDER | 0.34 | |
| | | Dimethicone-disodium stearoyl glutamate composite-treated black iron oxide | manufactured by Miyoshi Kasei, Inc., SA/NAI-B-10 MIBRID COLOR POWDER | 0.06 | |
| | | Dimethicone-disodium stearoyl glutamate composite-treated yellow iron oxide | manufactured by Miyoshi Kasei, Inc., SA/NAI-Y-10 MIBRID COLOR POWDER | 1.00 | |
| | | Amino acid-isopropyl triisostearoyl titanate composite-treated titanium oxide | manufactured by Ishihara Sanavo Kaisha Ltd., CR50 | | 8.800 |
| | | Amino acid-isopropyl triisostearoyl titanate composite-treated red iron oxide | | | 0.374 |
| | | Amino acid-isopropyl triisostearoyl titanate composite-treated black iron oxide | | | 0.066 |

(continued)

| | | Component name | Starting material name | Example 8 | Example 9 |
|---|---|---|---|---|---|
| | | Amino acid-isopropyl triisostearoyl titanate composite-treated yellow iron oxide | | | 1.100 |
| | (D) | Silicic anhydride | manufactured by Momentive Performance Materials Japan LLC, HARMONIE LUXE 4 POWDER (specific surface area: 1.3 $m^2/g$) | 3.00 | 5.00 |
| | (D) | Dimethicone-treated silicic anhydride | manufactured by Miyoshi Kasei, Inc., MivoFEEL SA-SB-N1 (specific surface area: 80 $m^2/g$) | 2.00 | |
| | Other components | Chromium hydroxide | manufactured by Sensient Cosmetic Technologies SAS, UNIPURE GREEN LC 790 CF | 0.10 | 0.10 |
| | Other components | Amino acid-isopropyl triisostearoyl titanate composite-treated titanated mica | manufactured by Merck KGaA, LUMINOUS GLOW | 3.70 | 3.70 |
| | Other components | Polymethylsilsesquioxane | manufactured by Momentive Performance Materials Japan LLC, TOSPEARL 145A | | |
| Total | | | | 100.000 | 100.000 |
| Total (A) | | | | 14.44 | 15.38 |
| Total (B) | | | | 0.50 | 0.50 |
| Total (C) | | | | 4.00 | 4.00 |
| Total (D) | | | | 3.00 | 5.00 |
| Total (E) | | | | 12.00 | 12.10 |
| (A)/(D) | | | | 4.81 | 3.08 |
| (B)/(D) | | | | 0.17 | 0.10 |
| (C)/(D) | | | | 1.33 | 0.80 |

## Claims

**1.** A water-in-oil emulsion composition for impregnation comprising the following components (A), (B), (C), (D), and (E) :

(A) a hydrophobized color pigment,
(B) an acrylic silicone polymer,
(C) one or more members selected from the group consisting of polyether-modified silicone and polyglyceryl ether-modified silicone,
(D) spherical silica, and
(E) an ester oil which is in a liquid state at 25°C.

**2.** A cosmetic in which a holder impregnated with the water-in-oil emulsion composition according to claim 1 is housed in a compact container.

3. The cosmetic according to claim 2, wherein the hydrophobization treatment of the component (A) comprises one or more members selected from the group consisting of amino acid treatment, acylated amino acid treatment, and alkoxysilane treatment.

4. The cosmetic according to claim 2 or 3, wherein the component (B) is an (acrylates/ethylhexyl acrylate/dimethicone methacrylate) copolymer.

5. The cosmetic according to any one of claims 2 to 4, wherein the spherical silica as the component (D) has a specific surface area of 150 $m^2/g$ or less.

6. The cosmetic according to any one of claims 2 to 5, wherein a content of the component (A) is from 1 to 30% by mass, a content of the component (B) is from 0.01 to 5% by mass, a content of the component (C) is from 0.1 to 12% by mass, a content of the component (D) is from 0.1 to 20% by mass, and a content of the component (E) is from 3 to 40% by mass.

7. The cosmetic according to any one of claims 2 to 6, wherein a mass ratio of the component (A) to the component (D), (A)/(D), is from 0.3 to 15.

8. The cosmetic according to any one of claims 2 to 7, wherein a mass ratio of the component (B) to the component (D), (B)/(D), is from 0.01 to 1.

9. The cosmetic according to any one of claims 2 to 8, wherein a mass ratio of the component (C) to the component (D), (C)/(D), is from 0.2 to 5.

10. The cosmetic according to any one of claims 2 to 9, wherein a viscosity at 25°C of the component (E) is 10 000 mPa·s or less.

11. The cosmetic according to any one of claims 2 to 10, wherein the water-in-oil emulsion composition further comprises from 1 to 50% by mass of a volatile oil.

12. The cosmetic according to any one of claims 2 to 11, wherein the water-in-oil emulsion composition further comprises from 1 to 15% by mass of alkyl-modified silicone which is in a liquid state at 25°C.

13. The cosmetic according to any one of claims 2 to 12, wherein a content of water in the water-in-oil emulsion composition is from 3 to 50 by mass.

14. The cosmetic according to any one of claims 2 to 13, wherein a viscosity at 30°C of the water-in-oil emulsion composition is from 3 000 to 30 000 mPa·s.

15. The cosmetic according to any one of claims 2 to 14, wherein the holder is a foam, a porous material, or an assembly of fibers.

16. The cosmetic according to any one of claims 2 to 15, wherein the holder is an assembly of polyester fibers.

17. A method for using the cosmetic according to any one of claims 2 to 16, comprising taking a water-in-oil emulsion composition impregnated into a holder by finger or an applicator from the holder, and applying it to the skin.

18. Use of a water-in-oil emulsion composition comprising the following components (A), (B), (C), (D), and (E):

(A) a hydrophobized color pigment,
(B) an acrylic silicone polymer,
(C) one or more members selected from the group consisting of polyether-modified silicone and polyglyceryl ether-modified silicone,
(D) spherical silica, and
(E) an ester oil which is in a liquid state at 25°C as a makeup cosmetic comprising a holder impregnated with the water-in-oil emulsion composition.

19. A cosmetic kit comprising a set of a holder impregnated with a water-in-oil emulsion composition, and a compact container, the water-in-oil emulsion composition comprising the following components (A), (B), (C), (D), and (E):

(A) a hydrophobized color pigment,
(B) an acrylic silicone polymer,
(C) one or more members selected from the group consisting of polyether-modified silicone and polyglyceryl ether-modified silicone,
(D) spherical silica, and
(E) an ester oil which is in a liquid state at 25°C.

**20.** A water-in-oil emulsion composition comprising the following components (A), (B), (C), (D), and (E):

(A) a hydrophobized color pigment,
(B) an acrylic silicone polymer,
(C) one or more members selected from the group consisting of polyether-modified silicone and polyglyceryl ether-modified silicone,
(D) spherical silica, and
(E) an ester oil which is in a liquid state at 25°C.

## INTERNATIONAL SEARCH REPORT

International application No.
**PCT/JP2024/028457**

**A. CLASSIFICATION OF SUBJECT MATTER**

***A61K 8/29***(2006.01)i; ***A61K 8/06***(2006.01)i; ***A61K 8/19***(2006.01)i; ***A61K 8/37***(2006.01)i; ***A61K 8/46***(2006.01)i; ***A61K 8/81***(2006.01)i; ***A61K 8/89***(2006.01)i; ***A61K 8/893***(2006.01)i; ***A61Q 1/02***(2006.01)i
FI: A61K8/29; A61K8/06; A61K8/19; A61K8/37; A61K8/46; A61K8/81; A61K8/89; A61K8/893; A61Q1/02

According to International Patent Classification (IPC) or to both national classification and IPC

**B. FIELDS SEARCHED**

Minimum documentation searched (classification system followed by classification symbols)

A61K8/29; A61K8/06; A61K8/19; A61K8/37; A61K8/46; A61K8/81; A61K8/89; A61K8/893; A61Q1/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2007-119741 A (SHIN-ETSU CHEMICAL CO., LTD.) 17 May 2007 (2007-05-17) claims 1-32, paragraphs [0022]-[0031], example 50 | 1-19 |
| Y | JP 3227541 U (KOLMAR KOREA CO., LTD.) 03 September 2020 (2020-09-03) claims 1-10, paragraphs [0001], [0027]-[0028], example 4 | 1-19 |
| Y | JP 2023-098745 A (KOSE CORP.) 11 July 2023 (2023-07-11) claims 1-9, example 37 | 4-17 |
| A | WO 2019/107497 A1 (SHIN-ETSU CHEMICAL CO., LTD.) 06 June 2019 (2019-06-06) entire text, all drawings | 1-19 |
| A | JP 2011-088832 A (SHIN-ETSU CHEMICAL CO., LTD.) 06 May 2011 (2011-05-06) entire text, all drawings | 1-19 |
| A | JP 2009-263213 A (SHIN-ETSU CHEMICAL CO., LTD.) 12 November 2009 (2009-11-12) entire text, all drawings | 1-19 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 October 2024** | **29 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/028457**

**C. DOCUMENTS CONSIDERED TO BE RELEVANT**

| Category* | Citation of document, with indication, where appropriate, of the relevant passages | Relevant to claim No. |
|---|---|---|
| A | WO 2020/157956 A1 (SHISEIDO COMPANY, LTD.) 06 August 2020 (2020-08-06) entire text, all drawings | 1-19 |
| A | JP 2019-112330 A (NIPPON MENAADE KESHOHIN KK) 11 July 2019 (2019-07-11) entire text, all drawings | 1-19 |
| A | WO 2011/102123 A1 (CHIBA FLOUR MILLING CO., LTD.) 25 August 2011 (2011-08-25) entire text, all drawings | 1-19 |

Form PCT/ISA/210 (second sheet) (July 2022)

**INTERNATIONAL SEARCH REPORT**

International application No.
**PCT/JP2024/028457**

**Box No. III Observations where unity of invention is lacking (Continuation of item 3 of first sheet)**

This International Searching Authority found multiple inventions in this international application, as follows:

Document 1: JP 2007-119741 A (SHIN-ETSU CHEMICAL CO., LTD.) 17 May 2007 (2007-05-17) claims 1-32, example 50

Claims are classified into the following two inventions.

(Invention 1) Claims 1-19

Document 1 discloses (claims 1-32, example 50) a water-in-oil emulsion composition containing organopolysiloxane-treated zinc oxide (corresponding to component (A) of the present invention), an acrylic silicone resin (corresponding to component (B) of the present invention), a crosslinked polyether-modified silicone, an alkyl-polyether covalent branched type silicone and an alkyl-polyglycerin-modified branched silicone (each corresponding to component (C) of the present invention), silica, and dioctyl neopentyl glycol and ethylhexyl methoxycinnamate (each corresponding to component (E) of the present invention). Accordingly, the invention in claims 1-17 of the present application has the special technical feature of "impregnation" and is thus classified as invention 1.

In addition, the inventions in claims 18 and 19 specify "impregnating a water-in-oil emulsion composition in a holding body" and including "a holding body impregnated with a water-in-oil emulsion composition," respectively, and are substantially identical to or similarly closely related to claim 1, which is a "water-in-oil emulsion composition for impregnation," and are thus classified as invention 1.

(Invention 2) Claim 20

Since claim 20 does not specify that the water-in-oil type emulsion composition is for "impregnation," claim 20 cannot be said to have a special technical feature identical or corresponding to that of claim 1 classified as invention 1.

Thus, claim 20 cannot be classified as invention 1, and is thus classified as invention 2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-19**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/JP2024/028457**

| Patent document cited in search report | Publication date (day/month/year) | Patent family member(s) | Publication date (day/month/year) |
|---|---|---|---|
| JP 2007-119741 A | 17 May 2007 | US 2007/0071980 A1<br>claims 1-32, paragraphs [0041]-[0057], example 50<br>EP 1772138 A2 | |
| JP 3227541 U | 03 September 2020 | US 2023/0240428 A1<br>claims 1-10, paragraphs [0001], [0052], example 4<br>WO 2019/017737 A1<br>CN 210055027 U | |
| JP 2023-098745 A | 11 July 2023 | (Family: none) | |
| WO 2019/107497 A1 | 06 June 2019 | US 2020/0332065 A1<br>EP 3719056 A1<br>CN 111433256 A | |
| JP 2011-088832 A | 06 May 2011 | (Family: none) | |
| JP 2009-263213 A | 12 November 2009 | US 2009/0252774 A1<br>EP 2107080 A1 | |
| WO 2020/157956 A1 | 06 August 2020 | CN 111801092 A | |
| JP 2019-112330 A | 11 July 2019 | (Family: none) | |
| WO 2011/102123 A1 | 25 August 2011 | US 2012/0316332 A1<br>EP 2537865 A1<br>CN 102753583 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013530252 A
- JP 2017088631 A
- JP 2019131489 A
- JP 11001530 A
- JP 2000063225 A
- JP 4134013 A
- JP 4195043 B